# EUROPEAN PATENT SPECIFICATION

(11) **EP 4 489 701 B1**
(45) Date of publication and mention of the grant of the patent: **26.11.2025**
(21) Application number: 23701844.5
(22) Date of filing: 11.01.2023
(51) Int. Cl.: A61F 9/00

(54) **DELIVERY SHAFT ASSEMBLY FOR DELIVERY OF AN IMPLANT**
EINFÜHRUNGSSCHAFTANORDNUNG ZUR EINFÜHRUNG EINES IMPLANTATS
ENSEMBLE ARBRE DE MISE EN PLACE D'UN IMPLANT

(30) Priority: 09.03.2022 EP 22161176
(43) Date of publication of application: 15.01.2025
(73) Proprietor: iSTAR Medical SA, 1300 Wavre (BE)
(72) Inventor: UHRLANDT, Stefan, 22587 Hamburg (DE); SEDANO SANTOS, Elena, 28039 Madrid (ES); DETRY, Benoit, 4432 Alleur (BE); CLEMENCE, Adrien, 1300 Wavre (BE)
(74) Representative: De Clercq & Partners
(86) International application number: PCT/EP2023/050508
(87) International publication number: WO 2023/169720

(56) References cited:
- WO-A1-2016/159999

## Description

### Field of the invention

Provided herein is a delivery shaft assembly for delivery of an occur implant, for implantation in a subject.

### Background to the invention

Implants are often used to treat conditions where medicinal treatment has become ineffective or is not available. Techniques for placement of an implant typically rely on an inserter tool that temporarily holds the implants and provides an elongated reach for placement at the implantation site and a mechanism for ejection from the inserter. Implants can be very small, for instance, implants for the treatment of glaucoma typically have a length of 5 mm and a width of 1 mm. An example of an implant and inserter tool for treatment of glaucoma is disclosed, for instance, in WO 2017/108498 and in WO 2016/159999.

A problem in the art is how to introduce and deploy the implant with an introducer that is minimally traumatic, yet offers sufficient pushing force and flexibility to enter into the curved tissue of the eye. A further problem is visibility of the implant during surgery; the implant being concealed within an introducer requires a solution for visibility under light microscopy before and during deployment.

### Summary

Provided is a delivery shaft assembly (200) having a proximal end (20) and a distal end (40) for delivery of an ocular implant (230), wherein:
- delivery shaft assembly (200) comprises a delivery shaft (220) having a lumen (222) configured for holding the implant (230), and an adapter (210) at the proximal end (20) of the delivery shaft (220) configured for attachment to an inserter tool (500) for deployment of the implant (230);
- the delivery shaft (220) comprises a distal compliant section (244) that is repeatably bendable, compliant, and biased in a curve in a 1^{st} plane (60);
- the delivery shaft (220) is provided with an axially longitudinal observation fenestration (290) at least partly disposed in the distal compliant section (244) allowing visualisation of the implant (230) from an anterior (52) side of the delivery shaft (220);
- the delivery shaft (220) comprises a distal tip section (246) having an atraumatic distal tip,
- a wall (224) of the tube (250) in the distal compliant section (244) is provided with a plurality of flexibility slots (252) that impart the bendability,
- the wall (224) of the tube (250) in the distal tip section (246) comprises a pair of cut outs (270-a, 270-b) that define a restricting flap (272) and an extension body (274), wherein, the restricting flap (272) and extension body (274) co-operate to form a wedge-shaped distal tip, and the restricting flap (272) is moveable around a living hinge (276) between an open state and an occluding state and is biased in the occluding state.

The observation fenestration (290) may be defined by an inner edge (292) that is continuous.

The observation fenestration (290) may be surrounded by a border (294) that is a region of the delivery shaft (220) tube (250) wall around the observation fenestration (290), wherein the border is continuous.

The border (294) may have two opposing long sides (294-a, 294-b), and each border long side (294-a, 294-b) in the distal compliant section (244) gradually decreases in width towards the distal end (40) of the distal compliant section (244).

The observation fenestration (290) may be continuous and spans at least a part of the distal compliant section (244) and of the distal tip section (246).

The delivery shaft assembly (200) according to any one of claims 1 to 5, wherein the plurality of flexibility slots (252) are spaced apart in at least a longitudinal direction.

Each flexibility slot (252-a; 252-b) of the plurality of flexibility slots (252) may have a net slot path (282) containing one or more inclined portions (284), and a pair of longitudinally adjacent flexibility slots (252-a; 252-b) each contain one or more inclined portions (284-a; 284-b), and a plane perpendicular to a central longitudinal axis (a-a') of the tube (250) crosses both longitudinally adjacent flexibility slots (252-a; 252-b).

A radial perimetric integrity may be equal to or greater than 50% at all positions along the longitudinal length of the distal compliant section (244), wherein the radial perimetric integrity is a proportion of a perimeter length of wall material present in a plane perpendicular to the central axis (a-a') of the tube (250) in the distal compliant section (244), compared with a length of the perimeter (*pl*) of the intact tube (250) in the same plane.

The net slot path (282) of each flexibility slot (252) in the distal compliant section (244) may contain two inclined portions (284-a, 284-b) connected on the posterior side (54) of the tube (250), preferably by a perpendicular portion (286), and each flexibility slot (252) of the plurality has an identical net slot path (282).

Each inclined portion (284-a, 284-b) may extends toward the anterior side (52) of the delivery shaft (220) into a perpendicular portion (284-a, 284-b), and optionally each anterior side (52) perpendicular portion (284-a, 284-b) terminates in a rounded aperture (254-a, 254-b). An angle, alpha, of an incline of each inclined portion (284-a, 284-b) may be the same and is 55 to 65 deg.

A distance (sd) between adjacent flexibility slots (252) may be the same for the flexibility slots (252) of plurality, and is a value in a range 0.20 to 0.30 mm.

The plurality of flexibility slots (252) in the distal compliant section (244) may be arranged into a set of narrower flexibility slots (252-e₁, 282-e₁, 252-e₂, 282-e₂) and a set of wider flexibility slots (252-f, 282-f),
- the narrower (252-e₁, 282-e₁, 252-e₂, 282-e₂) and wider (252-f, 282-f) flexibility slots may alternate and be spaced apart in longitudinal direction,
- each wider flexibility slots 252-f, 282-f) may contain one continuous inclined portion (284-f), and
- each narrower flexibility slot (252-e₁, 282-e₁, 252-e₂, 282-e₂) may contain two continuous inclined portions (284-e₁, 284-e₂), spaced apart by an intact section (283-e) of tube (250) wall on the posterior side (54) of the tube (250).

An angle, alpha, of an incline of each inclined portion (284-e₁, 284-e₂, 284-f) may be the same and may be 55 to 65 deg.

The curve of the distal compliant section (244) may contain a radius of between 30 to 50 mm.

### Figure Legends

**FIG. 1** view of a delivery shaft assembly as described herein coupled to an inserter tool. In Panel A prior to deployment, the implant is held in the delivery shaft lumen. In Panel B after deployment, the delivery shaft is withdrawn proximally over the discharge shaft and the implant is released.
**FIG. 2** is a side lateral view of a delivery shaft assembly as described herein
**FIG. 2A** is a plan view of a part of the delivery shaft assembly of FIG. 2.
**FIG. 2B** is a transverse-cross-sectional view of the delivery shaft assembly of FIG. 2 through plane A-A'.
**FIG. 2C** is a transverse-cross-sectional view of the delivery shaft assembly of FIG. 2 through plane B-B'.
**FIG. 2D** is a transverse-cross-sectional view of the delivery shaft assembly of FIG. 2 with the perimeter and perimeter length (*pl*) indicated.
**FIG. 2E** is a flattened (net) view of a flexibility slot, with the slot path length (*sl*) indicated.
**FIG. 3** is a plan view of an implant described herein.
**FIG. 3A** is a transverse cross-section of the implant of FIG. 3 through plane C-C'.
**FIG. 4A** shows detail of an incline portion of flexibility slot on a tube wall.
**FIG. 4B** shows detail of an incline portion of flexibility slot comprising and terminating in a rounded aperture.
**FIG. 4C** shows detail of an observation fenestration a tube wall.
**FIG. 5** is a net representation of the distal compliant section and distal tip section of the delivery shaft/tube wall. Exemplified is a snake formation of flexibility slots.
**FIG. 6** is a net representation of a detail of portions of a slot path of the net representation of FIG. 5.
**FIG. 7** is a net representation of a detail of an inclined portion of a slot path of the net representation of FIG. 5, with angle alpha and slot width (sw) indicated.
**FIG. 8** is a net representation two slot paths of FIG. 5, with indicated spacing.
**FIG. 9** is a net representation of two slot paths of FIG. 5, with path crossing a perpendicular axis.
**FIG. 10** is a net representation of the distal compliant section and distal tip section of the delivery shaft/tube wall. Exemplified is a shark formation of flexibility slots.
**FIG. 10A** is a net representation of a detail of portions of a slot path of the net representation of FIG. 10.
**FIG. 10B** show a detail of flexibility slots of FIG. 10A, with the slot path lengths (*sl₁ and sl₂*) indicated.
**FIG. 11** is a plan view (anterior-posterior) is an observation fenestration.
**FIG. 12** is a plan view (anterior-posterior) is an observation fenestration with border.
**FIG. 13** is a plan view (anterior-posterior) is an observation fenestration with border of gradually changing width in a distal direction.
**FIG. 14** depicts flexibility slots distal compliant section of gradually increasing length in a distal direction.
**FIG. 15A** and **15B** are photographs showing damage to the distal compliant section when the fenestration inner edge is not continuous and the border is absent.
**FIG. 16** is a photograph showing damage to the distal compliant section when radial perimetric integrity is below a threshold value.
**FIG. 17** demonstrates radial perimetric integrity. Indicated planar sections (A, B, C) through different axial positions of the distal compliant section of Panel D are presented in Panels A to C. Panels A1 to C1 show the corresponding intact parts of tube wall, allowing determination of the radial perimetric integrity which is indicted as a length percentage.
**FIG. 18** is an isometric view of a distal end of the delivery shaft, in which the restricting flap is in an occluding state.
**FIG. 19** is in an isometric view of a distal end of the delivery shaft, in which the restricting flap is in an open state.
**FIG. 20** is a detailed view of a restricting flap.
**FIG. 21** is a detailed view of an extension body.
**FIG. 22** is a side lateral view of the delivery shaft, with the slope angle indicated.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail.

Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal" or "distal to" and "proximal" or "proximal to" are used throughout the specification, and are terms generally understood in the field to mean towards (proximal) or away (distal) from the user's (practitioner's) side of an apparatus. Thus, "proximal" or "proximal to" means towards the user's side and, therefore, away from the subject's (patient's) side. Conversely, "distal" or "distal to" means towards the subject's side and, therefore, away from the user's side. The term "subject" refers to the human or animal receiving the implant. The term "user" refers to the person performing the implantation (e.g. surgeon, specialist, practitioner).

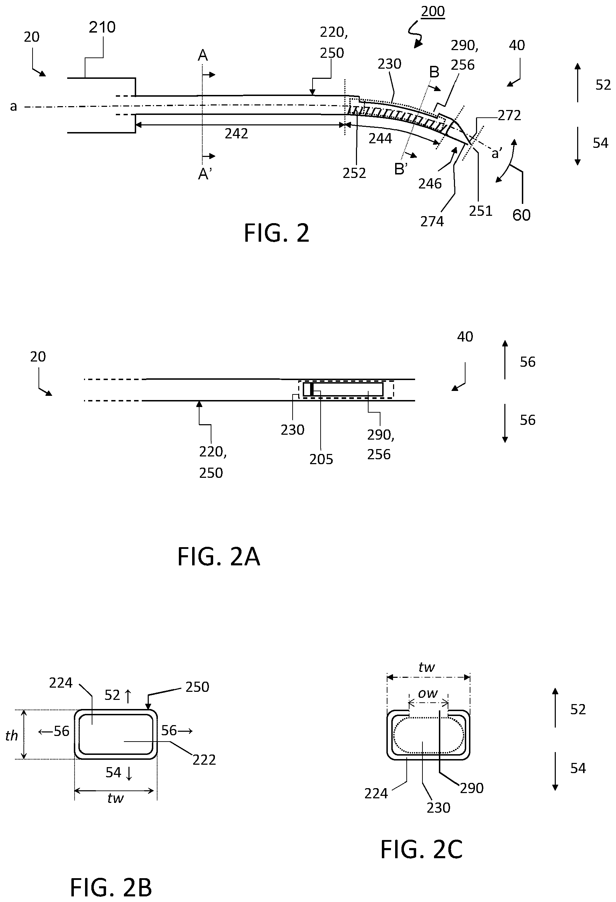

The terms "anterior ", "posterior", and "lateral" are used throughout the specification, and are terms generally understood in the field to mean the front side (anterior) of the subject, the rear-side (posterior) of the subject, and the sides (lateral) of the subject. **FIG. 2A** indicates anterior (52), posterior (54), and lateral (56) sides on a cross-section of the delivery shaft (250). The term "transverse" refers to direction across (*e.g*. perpendicular to) a longitudinal direction. The term "axial" refers to a longitudinal direction of the tube (250), e.g. along axis (a-a'). The term "perimetric" refers to a direction around the exterior of the tube (250), *e.g*. around perimeter (62).

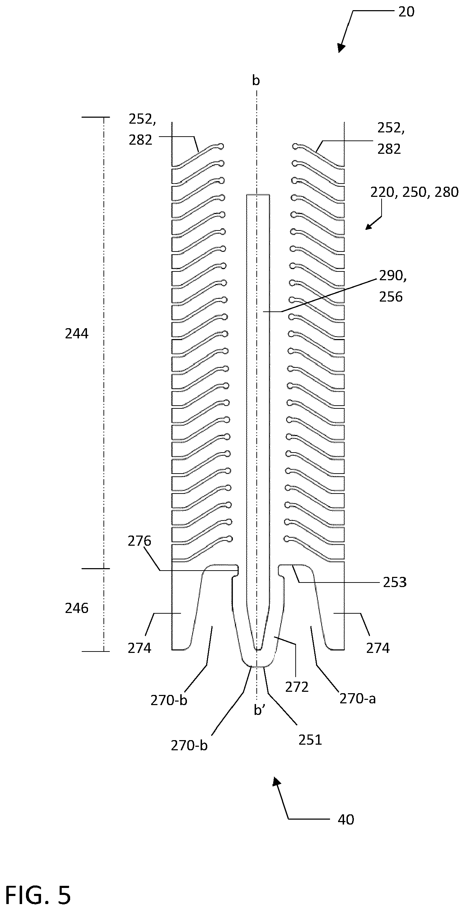

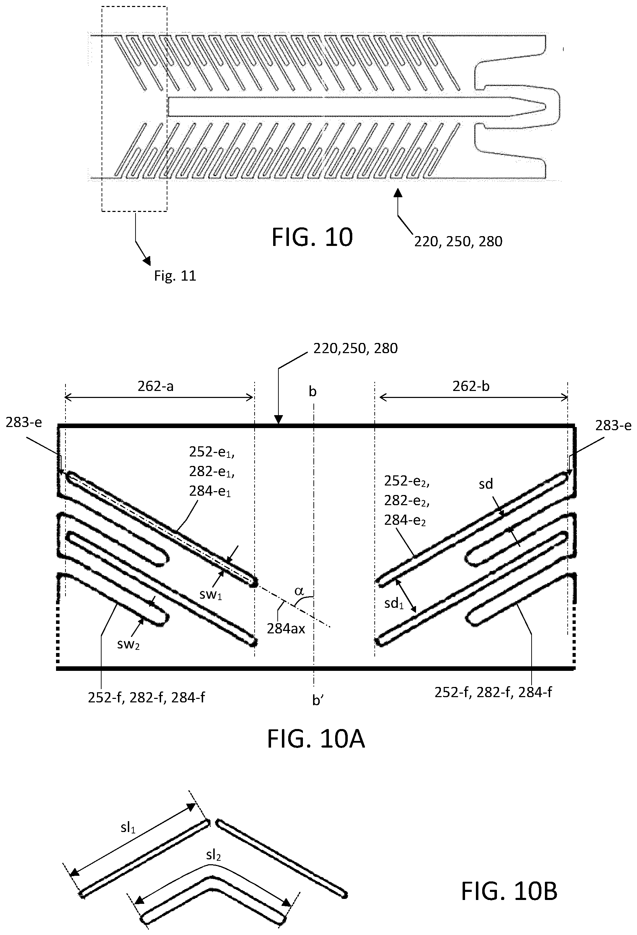

The tube (250) may be represented as a "net" (280). The net (280) is a two-dimensional representation that is known in the art for three dimensional objects. The net is obtained by unfolding the tube (250) along a fictive longitudinal cut and flattening it. A longitudinal axis (b-b') of the net (280) is parallel to a central axis (a-a') of the tube (250). Examples of the net (280) representation are shown in FIG. 5 and 10, showing the distal compliant section (244) and distal tip section (246).

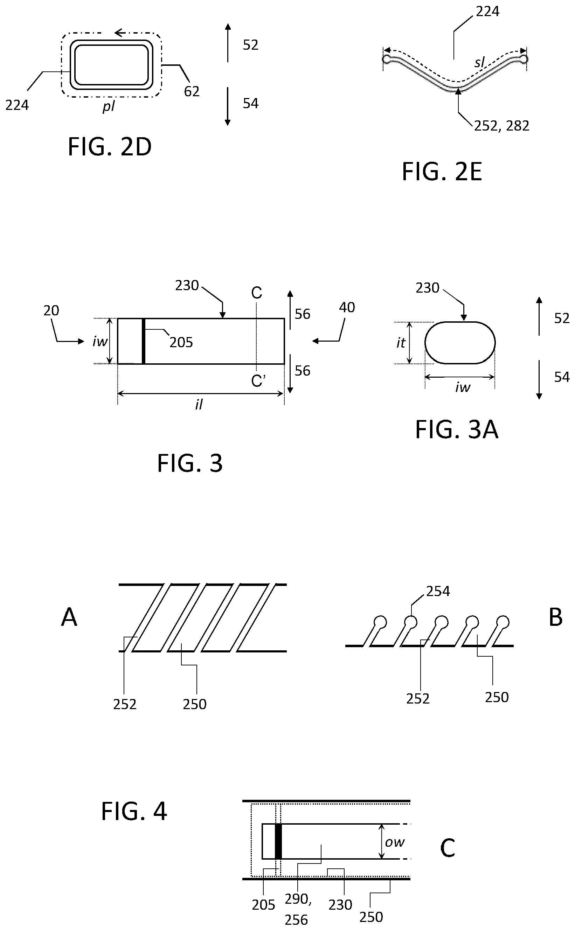

Provided herein is a delivery shaft assembly (200) for delivery of an ocular implant (230) as exemplified for instance in **FIG. 2****,** and a method for manufacture of the same. The delivery shaft assembly (200) has a proximal end (20) and a distal end (40) and comprises a delivery shaft (220) having a lumen (222) configured for holding the implant (230). The delivery shaft (220) comprises a distal compliant section (244) repeatably bendable (60). The distal compliant section (244) is biased in a curve. The delivery shaft (220) is provided with an observation fenestration (290) **(****FIG. 2A, FIG. 2C****)** allowing visualisation of the implant (230) within the delivery shaft (220) from the anterior (52) side by light that reflects from both the implant surface and through the body of a translucent implant. It can be visualised particularly well under light microscopy. The delivery shaft (220) comprises a distal tip section (246) having a restricting flap (272) and extension body (274). The distal compliant section (244) is connected to an adaptor (210) *via* a proximal section (242) of the delivery shaft (220). The proximal section (242) is straight, and may or may not contain one or more compliant portions. The delivery shaft (220) is formed from a compliant material (*i.e*. normally compliant as a sheet of that material) formed into a bend-resistive tube (250). A wall (224) of the tube (250) in the distal compliant section (244) is provided with a plurality of flexibility slots (252) that impart the bendability (60).

A method for manufacture of the delivery shaft assembly (200) comprises providing a bend-resistive tube (250), and forming the delivery shaft (220) by adding a plurality of flexibility slots (252) that impart the bendability. Other manufacturing steps are described elsewhere herein.

An advantage of the delivery shaft assembly (200) is a delivery shaft (220) having a pushability in an axial (proximal to distal) direction that allows the delivery shaft (220) to be pushed by the user without significant buckling, across the eye. The use of a bend-resistive tube (250) imparts a native stiffness for pushability. The bendability of the distal compliant section (244) allows the distal tip section (246) to follow the curvature of the eye, reducing application of forces caused by pushing to the suprachoroidal space, or subconjunctival space or intrascleral space. The bendability of the distal compliant section (244) allows the passage with reduced force of the straight and fixed discharge shaft (510) through the lumen (222) in the distal compliant section (244) so the implant can be deployed. The bendability of the distal compliant section (244) allows the passage of the straight and fixed discharge shaft (510) through the curved lumen (222) in the distal compliant section (244) so the implant can be deployed. The compliance of the distal compliant section (244) allows a reversible deformation of the curve in the distal compliant section (244) so that the distal tip section (246) can actively follow the different radius of curvature of the eye. As the radius of curvature changes during advancement, the advancing distal tip section (246) can reset its position to meet the new form of the advancing curvature. Closely following and adjusting to the tissue landscape reduces intraoperative bleeding.

An advantage of providing an observation fenestration (290) is improved visibility of the implant during deployment. A delivery shaft is typically made from a transparent polymeric material that allows the implant to be seen during insertion. However, because the transparent polymeric material needs to have good pushability, the wall thickness is such that, visibility of the implant or markers thereon is impaired. Increasing the intensity of the light source causes increased reflection which further impairs visualisation.

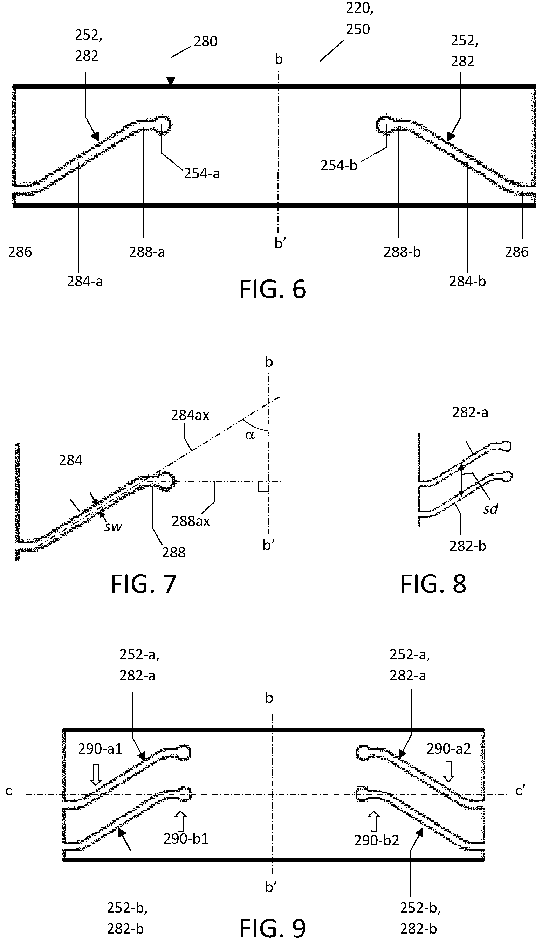

A flexibility slot (252) is a discrete slot in the wall (224) of the tube (250) that imparts bendability to the tube. A detail of a flexibility slot (252) is shown in **FIGs. 2E, 4A****,** **6** to **9** and **10A.** A path length of the slot (*sl*) **(****FIG. 2E****)** is greater than its width (sw) **(****FIG. 7****,** **10A****).** The path length (sl) is a distance around the outside of the tube between one closed end to another closed end of the flexibility slot (252). A flexibility slot (252) is preferably closed. In a closed flexibility slot (252) the internal edges of the slot form a continuous path. A closed flexibility slot (252) for instance does not open into the observation fenestration (290), thereby allowing the observation fenestration (290) to be defined by a continuous inner edge (292). As mentioned elsewhere herein, a continuous inner edge (292) prevents failure of distal compliant section (244) while uncurving forces are applied by the discharge shaft (510), leading to a more resilient delivery shaft.

A plurality of flexibility slots (252) is disposed in the distal compliant section (244). A plurality of flexibility slots (252) may or may not be disposed in the proximal section (242). Where there is a plurality of flexibility slots (252) in the proximal section (242), they may be disposed in a compliant portion of the proximal section (242).

One or both ends of a flexibility slot (252) may comprise and terminate in a rounded aperture (254) configured to reduce the bending force (see **FIG. 4B****).** The rounded aperture (254) may have a circular form. A majority of or all flexibility slots (252) are spaced apart at least in an axial direction.

Each flexibility slot (252) has a slot path, which describes the shape of the slot around the tube (250). The slot path may be described by reference to its three-dimensional shape, and/or by reference to its two-dimensional appearance of a net (280) of the tube (250). The "net slot path" is a path of a flexibility slot (252) described by its appearance on the net (280) of the wall of the distal compliant section (244). Examples of net representations are shown in **FIGs. 5** and **10****.**

A net slot path may contain one or more of:
- at least one "inclined portion" (*e.g*. **FIGs. 6, 7****,** 284), wherein an inclined portion is straight and disposed at an incline to the net (280) longitudinal axis (b-b');
- at least one "perpendicular portion" (*e.g*. **FIGs. 6, 7** 286, 288), wherein a perpendicular portion is a straight and disposed perpendicular to the net (280) longitudinal axis (b-b');
- at least one "parallel portion", wherein a parallel portion is a straight and disposed at parallel to the net (280) longitudinal axis (b-b');
- a rounded aperture (254) at one or both ends.
One or more of the above portions join together (*e.g*. end to end) to form a continuous net slot path.

The ocular implant (230) (also known as "implant" herein) may be any placeable in a subject (animal, human) using an inserter tool. In particular, the implant (230) may be an implant for treatment of glaucoma. In particular, the implant (230) may be an intraocular shunt. The implant (230) may be for implantation in the treatment target. The treatment target may be between the sclera and the choroid, *i.e*. in the suprachoroidal space, or between the conjunctiva and the sclera (subconjunctival space) or within the sclera (intrascleral space). The implant (230) be an implant as described in WO 2017/108498. In particular, the implant may be as described on page 13, lines 1 to page 14, line 5, and/or on page 15, line 27 to page 16 line 4, and/or on page 21, line 25 to page 22 line 21 of WO 2017/108498 which is incorporated herein by reference.

An exemplary implant (230) is depicted in **FIGs. 3** and **3A****.** It has a longitudinal form. It has a proximal (20) and distal (40) end. The long edges may be straight and parallel. One longitudinal end may be wider (*e.g*. 8 to 12 %) than the other opposing end. The long edges may be rounded. The proximal and distal ends may be planar and parallel. The edges of the ends may be rounded. A transverse cross section (C-C') **(****FIG. 3A****)** may be longitudinal. The implant (230) may have an oblong form in plan view. The implant (220) may have an oblong form in transverse cross-section view. In transverse cross-section view, the short sides may be rounded. The implant (230) may turn transparent or translucent when hydrated. The implant (230) may be opaque when non-hydrated and may become transparent or translucent when hydrated.

The implant may be disposed with one or more markers (205) provided thereon so that a user deploying the shunt or implant can control the depth of the implant in the treatment target with a portion thereof remaining in the anterior chamber. Placement of the shunt or implant within the eye is made using an implantation or deployment device. The shunt or implant (200) may have a marker (205) located in the proximity of its proximal end (20) so that it is possible to see its position through the observation fenestration (290). It will readily be appreciated that other markers (205) may be provided on the implant (200) for better visibility, for example, two markers may be provided at the proximal end (20) of the implant (200).

Typically, the implant (230) has a longitudinal length (*il*) of between 3mm and 9mm. The implant (230) may have a thickness (*it*) of between 0.3mm and 1mm. The implant (230) may have a width (*iw*) between 0.5mm and 2mm. In a preferred embodiment, the implant is 5mm long, 0.6mm thick and 1.1±0.2 mm wide. Dimension indicators are shown in **FIGs. 3** and **3A****.**

The implant (200) is located in the distal end (40) of the delivery shaft (240) prior to implantation. Preferably it is located at least partially in the distal compliant section (244). It may be located exclusively in the distal compliant section (244).

The implant may be made from a biocompatible material. The biocompatible material may be that such as that described in EP-B-2517619.

It will readily be appreciated that the implant may be made from other suitable biocompatible materials, for example, silicone. In EP-B-2517619, the described biocompatible material is porous and comprises a biocompatible polymer scaffold that defines an array of interconnected pores having similar diameters. Typically, the mean diameter of the pores is between about 20µm and about 90µm, preferably between about 25µm and about 75µm. For use in the implant of the present invention, the preferred range is between about 25µm and about 36µm.

The delivery shaft (220) (also referred to tube (250) herein) has a proximal (20) and distal (40) end. The delivery shaft (220) is disposed with an internal longitudinal lumen (222) open at both the proximal (20) and distal (40) ends. The lumen (222) in transverse (A-A', B-B') cross-section has a longitudinal profile **(****FIGs. 2B, 2C****)**. The lumen (222) in transverse (A-A', B-B') cross-section may have an oblong profile **(****FIG. 2B, 2C****);** the corners may be rounded. Transverse cross-sections of the lumen (222) may have a uniform size and shape along a proximal-distal direction in a compliant portion. Transverse cross-sections of the lumen (222) may have a uniform size and shape along a proximal-distal direction in the proximal section (242). Transverse cross-sections of the lumen (222) may have a uniform size and shape along a proximal-distal direction in the distal compliant section (244). The lumen (222) is configured for holding the implant (230).

The proximal (20) end of the delivery shaft (220) is attached to the adaptor (210). As shown in the figures, the proximal end may extend into a body of the adaptor to secure it and for stability.

The delivery shaft (220) is formed from a bend-resistive tube (250). By bend resistive, it is meant exhibiting little or no bending along an axial direction. Factors that contribute to bend-resistivity include wall thickness lumen size, and material of the tube. The skilled person will readily understand how to make a tube bend-resistive, and as a guidance, a tube made from a metal (*e.g*. pure metal, metal alloy, nitinol), having a maximum outer width of 0.96 mm and a wall thickness of 0.05 mm is bend-resistive. One or more flexibility slots (252) are added to the tube (250) in order to impart the bendability *e.g*. in the 1^{st} plane (60) **(****FIG. 2****)**, or to a range of planes including or centred on the 1^{st} plane.

The use of metal allows for a reduction of the outer diameter because metal has good manufacturability properties meaning that geometrical tolerances can be more accurately adjusted. A reduced diameter delivery shaft introduces less damage to the treatment target. Additionally, there is a reduction of the cyclodialysis cleft. Furthermore, a metallic tube can be sterilised *e.g*. by steam and/or immersion in saline under sterilisation conditions (*e.g*. 121 deg C for 30 minutes) and then stored in the saline solution. This reduces manufacturing costs as the delivery shaft (220) can be sterilised without loss of shape or function in the same vessel it will be sterilised and stored in.

The one or more flexibility slots (252) added to the tube (250) impart the bending property to the tube (250) that otherwise is bend resistive The material might be light opaque. Examples of suitable material include a metals such as nitinol.

As mentioned elsewhere, the delivery shaft (220) comprises a distal compliant section (244). The distal compliant section (244) may have a length between 8 to 90 %, preferably 10 to 35 % of the total exposed delivery shaft (220) length. The distal compliant section (244) is biased in a curve parallel to the 1st plane (60). The delivery shaft (220) further comprises a distal tip section (246).

One or more flexibility slots (252) and/or observation fenestration (290) and cut-outs (270-a, 270-b), and/or living hinge described elsewhere herein may be introduced into the tube (250) by any method for removing a region of the tube wall (224). Exemplary methods include laser cutting, photochemical etching, deep pressing, conventional chipping techniques such as drilling or milling, high pressure water jet cutting systems or any suitable material removing process available. Preferably laser cutting is used as this allows a very accurate and clean removal of material under reasonable economic conditions.

The delivery shaft (220) and the portions and sections are preferably formed from one piece of tube (250).

The delivery shaft (220) may have a length in an axial (a-a') direction of 30 mm to 45 mm, preferably 38 mm to 42 mm.

The length is measured along a central axis (a-a') from the proximal terminal end of the delivery shaft to the distal terminal end of the distal tip section (246). A few (0.5 to 6 mm) millimetres of the proximal end of the delivery shaft (220) is typically embedded in the distal end of the adaptor (210). The delivery shaft (240) may have a maximum outer tube width across the lateral sides (*tw* in **FIG. 2B****)** of 0.9 mm to 1.3 mm, preferably about 1.05 mm to 1.25 mm, more preferably 1.10±0.04 mm. The delivery shaft (240) may have an outer tube height across the posterior-anterior side of (*th* in **FIG. 2B****)** of 0.5 mm to 0.9 mm, preferably about 0.6 mm to 0.8 mm, preferably about 0.70±0.04 mm.

As mentioned elsewhere herein, the distal compliant section (244) is compliant, repeatably bendable, and biased in a curve. The curve is in a posterior (54) direction. The curve may in a 1^{st} plane (60). The curve may have a path that is a circular segment. The curvature may not match the curvature of the eye as explained elsewhere herein.

The distal compliant section (244) may have a length along an axial (a-a') direction of 5 to 40 mm, preferably 5 to 12 mm. The length is measured along a central axis (a-a') from the distal end of the curve to the proximal end of the distal tip section (246).

The distal compliant section (244) may have a maximum outer tube width across the lateral sides (*tw* in **FIG. 2C****)** of 0.9 mm to 1.3 mm, preferably about 1.05 mm to 1.25 mm, more preferably 1.15±0.1 mm. The delivery shaft (240) may have an outer tube height across the posterior-anterior side of (*th* in **FIG. 2B****)** of 0.5 mm to 0.9 mm, preferably about 0.6 mm to 0.8 mm, preferably about 0.70±0.04 mm.

The delivery shaft (220) comprises a distal compliant section (244) repeatably bendable (60). The distal compliant section (244) is biased in shape containing a curve. The bendability of the distal compliant section (244) allows the curved distal compliant section (244) to repeatably curve more in order to adapt to the shape of the eye or to uncurve and open to a linear form (*i.e.* to straighten out). The distal compliant section (244) may bend or uncurve in only the 1^{st} plane. The opening to the linear form may be achieved by the application of an external force such as the fixed discharge shaft (510) or implant (230). The curve may contain (*e.g*. follow a circular segment with) a radius of between 30 to 50 mm, preferably 34 to 40 mm.

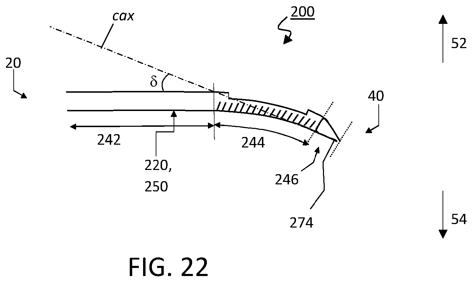

The curve of the distal compliant section (244) also defines a slope of the distal tip section (246). The slope is a measure of how much the distal tip section (246) is pointing in a posterior (54) direction (see **FIG. 22****).** The angle of the slope (delta) is an angle between a line (cax) that is parallel to and contacting the posterior (54) side of the extension body (246), wherein the line (cax) is also parallel to a central longitudinal axis of the tube (250), and the anterior side (52) of the tube (250) in the proximal section (242). Preferably, the slope (delta) is between 4 and 20 degrees in the curved state.

The curvature within this range does not match the curvature of the eye, but nonetheless, improves visualisation of the implant because it presents a flatter surface to the microscope compared with a curve of a smaller radius. In addition, a bigger diameter compared to the diameter of the eye reduces the risk of touching the iris or lens during passage across the eye, The compliance of the distal compliant section (244) still allows the curve to conform to that of the eye once the sheath is inside the tissues (supraciliary space). The curve within the range provides a natural tendency to the bending that eases the penetration in the supraciliary body but without hindering tissue targeting. The curve ensures that the distal tip section (246) is pointing in an optimal posterior (54) direction, and does not point too far down in the posterior (54) that would produce a kink upon application of pushing force.

As mentioned earlier a plurality of flexibility slots (252) is disposed in the distal compliant section (244). The number of flexibility slots (252) in the distal compliant section (244) may be 10 to 50. A width (sw) of a flexibility slot (252) in FIG. 7 may be 0.03 to 0.12 mm. A **distance (sd)** between adjacent flexibility slots (252) may be a value in the range 0.05 to 0.30 mm.

Each flexibility slot (252) has a slot path, which describes the shape of the slot around the tube (250) wall (224). The slot path may be described by reference to its three-dimensional shape, and/or by reference to its two-dimensional appearance of a net (280) of the tube (250). The "net slot path" (282) is a path of a flexibility slot (252) described by its appearance on the net (280) of the wall of the distal compliant section (244) as described earlier. In particular, the tubular wall of the distal compliant section (244) may be represented as a net (280). The net (280) is a two-dimensional representation that is known in the art for representing three dimensional objects. The net is obtained by unfolding the distal compliant section (244) along a fictive longitudinal cut in the tube and flattening it. A longitudinal axis (b-b') of the net (280) is parallel to a central axis of the distal compliant section (244). Examples of the net (280) representation is shown in **FIGs. 5** and **10****.**

In the distal compliant section (244), a net slot path may contain one or more of:
- at least one "inclined portion" (284, 284-a, 284-b), wherein an inclined portion (284-a, 284-b) is straight and disposed at an incline to the net (280) longitudinal axis (b-b');
- at least one "perpendicular portion" (286, 288-a, 288-b), wherein a perpendicular portion is a straight and disposed perpendicular to the net (280) longitudinal axis (b-b');
- at least one "parallel portion", wherein a parallel portion is a straight and disposed at parallel to the net (280) longitudinal axis (b-b').
One or more of the above portions join together (e.g. end to end) to form a continuous net slot path. Examples of inclined portion (284, 284-a, 284-b) and perpendicular portion (286, 288-a, 288-b) are shown in **FIG. 6****.**

Preferably, in the distal compliant section (244), a net slot path contains at least one inclined portion (284, 284-a, 284-b), wherein an inclined portion is straight and disposed at an incline to the net (280) longitudinal axis (b-b'), and one or more of
- at least one "perpendicular portion" (286, 288-a, 288-b), wherein a perpendicular portion is a straight and disposed perpendicular to the net (280) longitudinal axis (b-b');
- at least one "parallel portion", wherein a parallel portion is a straight and disposed at parallel to the net (280) longitudinal axis (b-b').
One or more of the above portions join together (*e.g*. end to end) to form a continuous net slot path.

When a portion of the net slot path (282) is inclined, it is meant that a central axis (284ax) of the inclined portion (284, 284-a, 284-b) extends and crosses a net (280) longitudinal axis (b-b') at an incline. This is exemplarily depicted in **FIGs. 7** and **10A****.** An incline means at an angle that is not 0 deg, nor 90 deg, nor 180 deg. The incline may have an angle alpha that is between 10 and 80 deg, preferably between 20 and 70 deg, preferably 55 to 65 deg .

When a portion of the net slot path (282) is perpendicular, it is meant that a central axis (288ax) of the perpendicular portion (286, 288-a, 288-b) extends and crosses a net (280) longitudinal axis (b-b') at 90 deg. This is exemplarily depicted in **FIG. 7****.**

A flexibility slot (252) may be separated longitudinally from its neighbouring flexibility slot (252) by a distance (*sd*). In other words, a net slot path (282-a) may be separated longitudinally from its neighbouring net slot path (282-b) by a distance (*sd*). This is exemplarily depicted in **FIG. 8****.** The value of sd may be in a range 0.20 to 0.3 mm.

The distance (*sd*) may be the same between a majority of pairs of flexibility slots (252) in the distal tip section (246). In other words, the distance (*sd*) may be the same between a majority of pairs of corresponding net slot paths (282-a, 282-b) in the distal tip section (246).

The distance (*sd*) may be the same between all pairs of flexibility slot (252) in the distal tip section (246).In other words, the distance (sd) may be the same between or all pairs of net slot paths (282-a, 282-b) in the distal tip section (246).

In addition to being separated longitudinally, adjacent flexibility slot (252) may be separated peripherally around the tube (250). In other words, at least some of the flexibility slots (252) may be preferably arranged in two or more rows (262-a, 262-b) extending in longitudinal direction. This is exemplified in **FIG. 10A****.** One rows (262-a) of flexibility slots (252-f, 282-f) may be positioned only within one radial segment, and another row (262-a) flexibility slots (252-f, 282-f) may be positioned only within another radial segment.

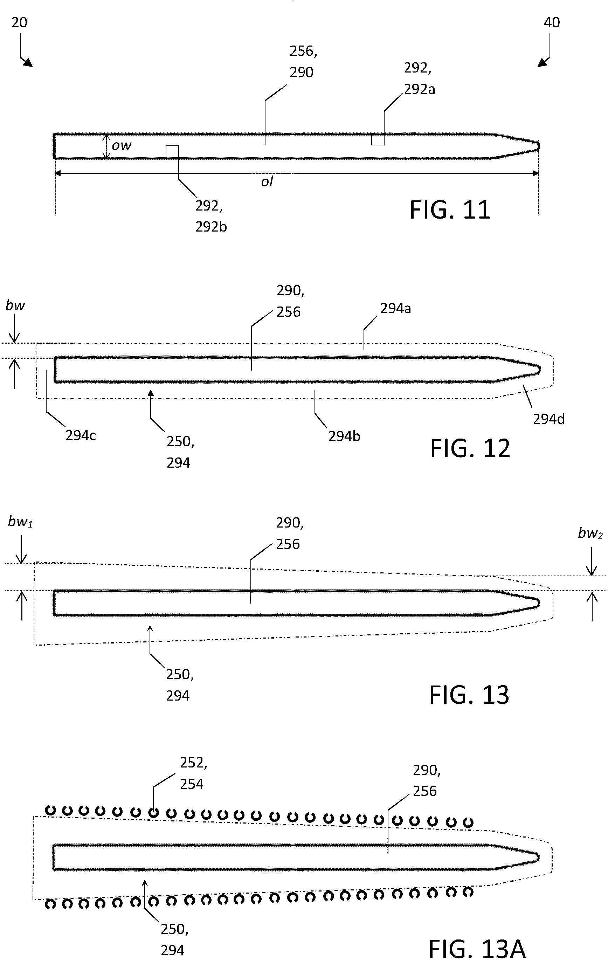

The plurality of flexibility slots (252) in the distal compliant section (244) may be arranged in a "snake formation". The snake formation is exemplified in **FIG. 5****.** In the snake formation, the net slot path (282) of each flexibility slot (252) in the distal compliant section (244) contains one or more inclined portions (284-a, 284-b), preferably two inclined portions. The flexibility slots are shown in **FIGs. 6 to 9****.** Where there are two inclined portions (284-a, 284-b), each spans at least a part (preferably all) of the lateral side (56) of the distal compliant section (244). The angle of the incline (alpha) of one inclined portion (284-a) may be 55 to 65 deg, and the angle of the incline (alpha) of the other inclined portion (284-b) may be 55 to 65 deg. Both angles of includes are preferably the same. Both inclined portions (284-a, 284-b) may be connected on the posterior side (54) of the tube (250), preferably by a perpendicular portion (286). Each inclined portion (284-a, 284-b) may extend towards the anterior side (52), into a perpendicular portion (288-a, 288-b). Each anterior side (52) perpendicular portion (288-a, 288-b) may terminate in a rounded aperture (254-a, 254-b). The rounded aperture (254-a, 254-b) may abut the observation fenestration (258) border (294) **(****FIG. 13A****).** In the snake formation, each and every flexibility slot (252) may have the same path shape, or be identical. In the snake formation, the plurality of flexibility slots (252) are spaced apart in longitudinal (axial) direction. A distance (sd) between adjacent flexibility slots (252) may be the same for flexibility slots (252) of plurality. The value of sd may be in a range 0.20 to 0.30 mm. The number of flexibility slots (252) in the snake formation may be between 25 and 35. A width (sw) (*e.g*. **FIG. 7****)** of a flexibility slot (252) in the snake formation may be 0.03 to 0.08 mm. A length (sl) (*e.g*. **FIG. 2E****)** of a flexibility slot may be 2 to 3mm.

The plurality of flexibility slots (252) in the distal compliant section (244) may be arranged in a "shark formation". The shark formation is exemplified in **FIG. 10****.** In the shark formation, there are two main sets of flexibility slots (252, 282), narrower (252-e₁, 282-e₁, 252-e₂, 282-e₂) and wider (252-f, 282-f). The flexibility slots are shown in **FIG. 10A****.** The narrower (252-e₁, 282-e₁, 252-e₂, 282-e₂) and wider (252-f, 282-f) flexibility slots alternate and are spaced apart in longitudinal (axial) direction. A distance (sd) between longitudinally adjacent flexibility slots (252) in the shark formation may be the same for flexibility slots (252) of plurality. The value of sd may be in a range 0.05 to 0.30 mm . In **FIG. 10A****,** distance (sd) between adjacent flexibility slots (252) (narrower to wider) is indicated; the value of sd may be in a range 0.05 to 0.1 mm. In **FIG. 10A****,** distance (sd₁) adjacent narrower flexibility slots (252) (narrower to narrow) is indicated; the value of sd₁ may be in a range 0.25 to 0.3 mm.

A width (sw₁) of a narrower flexibility slot (252-e₁, 282-e₁, 252-e₂, 282-e₂) is less than that of a wider flexibility slots (252-f, 282-f). A width (sw ₁) of a narrower flexibility slot (252-e₁, 282-e₁, 252-e₂, 282-e₂) may be 0.03 to 0.08 mm. A width (sw₂) of a wider flexibility slot (252, 252-f, 282-f) may be 0.08 to 0.12 mm.

In the shark formation, the net slot path (282-e₁, 282-e₂) of each narrower flexibility slot (252-e₁, 252-e₂) contains two continuous inclined portion (284-e₁, 284-e₂), spaced apart by an intact section (283-e) of tube (250) wall on the posterior side (54) of the tube (250). Each of the two continuous inclined portions (284-e₁, 284-e₂) is disposed at least partially on the lateral side (56) of the tube (250). A length (sl₁) (*e.g*. **FIG. 10B****)** of each continuous inclined portion (284-e₁, 284-e₂) of the narrower flexibility slot (252-e₁, 252-e₂) slot may be 2 to 3 mm

In the shark formation, the net slot path (282-f) of each wider flexibility slot (252-f) in the distal compliant section (244) contains one continuous inclined portion (284-f); the one continuous inclined portion (284-f) is disposed at least partially on the posterior side (54) of the tube (250). A length (sl₂) (e.g. **FIG. 10B****)** of each continuous inclined portion (284-f) of the wider flexibility slot (252-f) slot may be 0.7 to 1.24 mm.

The angle of the incline (alpha) of each inclined portion (284-e₁, 284-e₂) of the narrower flexibility slot (252-e₁, 282-e₁, 252-e₂, 282-e₂) may be 55 to 65 deg. The angle (alpha) of the incline of inclined portion (284-f) of the wider flexibility slot (252-f) may be 55 to 65 deg. The angle of the each inclined portion (284-e₁, 284-e₂) of the narrower flexibility slot (252-e₁, 282-e₁, 252-e₂, 282-e₂) and the angle of the incline of inclined portion (284-f) of the wider flexibility slot (252-f, 282-f) may be the same. The angle alpha is marked on the narrower flexibility slot (252-e₁, 282-e₁) of **FIG. 10A****.**

The number of narrower flexibility slot (252-e₁, 282-e₁, 252-e₂, 282-e₂) in the shark formation may be between 15 and 25 (one narrower flexibility slot contains two inclined portions (252-e₁, 282-e₁ and 252-e₂, 282-e₂). The number of wider flexibility slot (252, 252-f, 282-f) in the shark formation may be between 15 and 25.

Each end of the narrower flexibility slot (252-e₁, 282-e₁, 252-e₂, 282-e₂) and/or of each wider flexibility slot (252-f, 282-f) may or may comprise a terminal rounded aperture (254). Preferably each narrower flexibility slot (252-e₁, 282-e₁, 252-e₂, 282-e₂) has an identical slot path. Preferably each wider flexibility slot (252-f, 282-f) set has an identical slot path.

In a preferred configuration, a pair of adjacent flexibility slot (252-a; 252-b) each contain one or more inclined portions (284-a; 284-b), and a plane perpendicular to the longitudinal axis (a-a') crosses both adjacent flexibility slots (252-a; 252-b). In a preferred configuration, a pair of adjacent net slot paths (282-a; 282-b) each contain one or more inclined portions (284-a; 284-b), and an axis perpendicular (c-c') to the net (280) longitudinal axis (b-b') (the net (280) perpendicular axis (c-c')) crosses both net slot paths (282-a; 282-b). This is exemplarily depicted in **FIG. 9****.**

As shown in **FIG. 9****,** a pair of adjacent net slot paths (282-a; 282-b) each contain one or more inclined portions (284-a; 284-b), and an axis perpendicular (c-c') to the net (280) longitudinal axis (b-b') (the net (280) perpendicular axis (c-c')) crosses both net slot paths (282-a; 282-b) at points 290-a1, 290-a2, 290-b1, 290-b2. The ability of the net (280) perpendicular axis (c-c') to cross at least two net slot paths is as a result of the one or more inclined portions present in each net slot path, and of the distance (sd) between the flexibility slots (252) (or between corresponding net slot paths (282-a)).

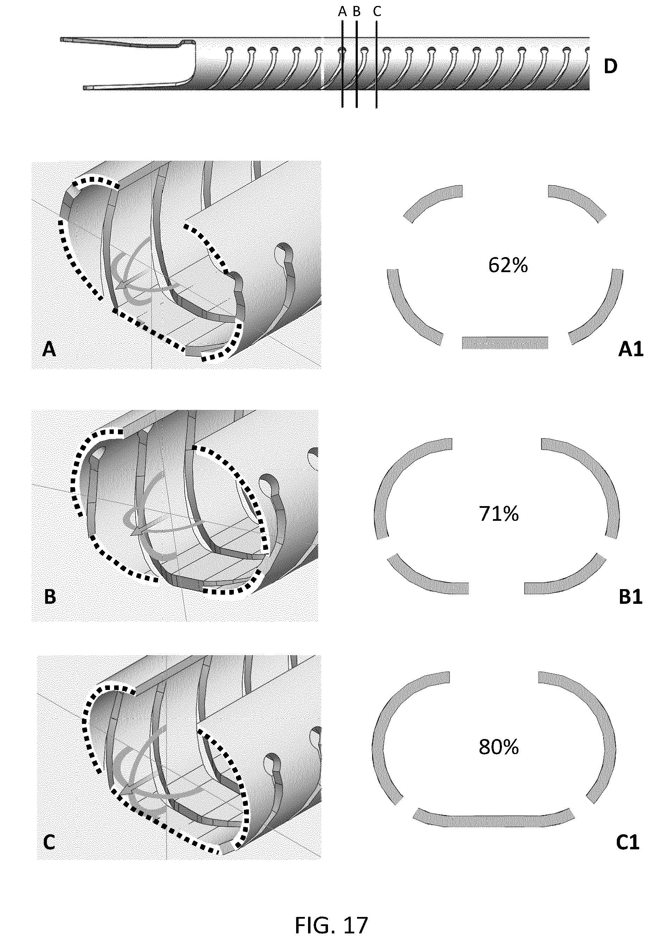

The plurality of discrete slots (252) in the distal compliant section (244) may be arranged such that the radial perimetric integrity is greater than or equal to a threshold value that is 50%, preferably greater than or equal to 60%. Preferably, the radial perimetric integrity is greater than or equal to a threshold value at any position (*i.e.* at all positions) along the longitudinal length of the distal compliant section (244). An upper limit to the threshold value may be 90%, preferably 85%.. The radial perimetric integrity is a proportion of a perimeter length of wall material present in a plane perpendicular to the central axis (a-a') of the tube (250) in the distal compliant section (244), compared with a length of the perimeter (*pl*) of the intact tube (250) in the same plane. **FIG. 17** depicts cross-sections at three different planes (A, B, C) perpendicular to the central axis (a-a') of the central axis (a-a') of the tube (250) in the distal compliant section (244) where the plurality of flexibility slots (252) are arranged in a snake formation. Each of panels A to C show the wall material present (dotted lines) on a three-dimensional model of the tube (250) at the three different planes (A to C), and panels A1 to C1 show two-dimensional cross-section at the three different planes (A to C), and the determined radial perimetric integrity expressed as a percentage. The radial perimetric integrity varies as a function of longitudinal position, and it remains equal to or above 60%. The radial perimetric integrity is determined by the distance (sd) between adjacent flexibility slots (252) and by the slot path which typically contained contains at least one inclined portion (284, 284-a, 284-b).

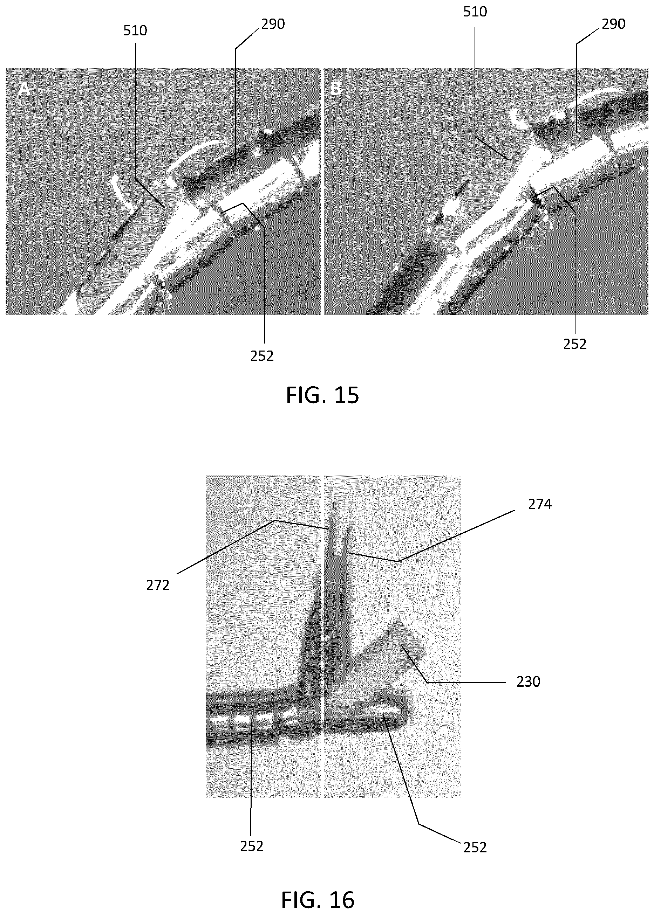

The inventors have found that when the plurality of discrete slots in the distal compliant section (244) have radial perimetric integrity equal to or greater than the threshold value at any position (*i.e.* at all positions) along the longitudinal length of the distal compliant section (244), the integrity of the distal compliant section (244) is maintained during deployment of the implant. Before reaching the target tissue, the implant is compressed inside the lumen. During deployment, passage through the lumen (222) becomes impeded because of high friction. When the friction is too high, the flexibility slots open too far, causing the discharge shaft (510) to pass through the wall of the tube (250), causing mechanical failure as shown in **FIG. 16****.** To solve the problem, it was found that radial perimetric integrity needs to be above a certain threshold; where positions exist in which the radial perimetric integrity falls below the threshold, for instance where all the flexibility slots are disposed perpendicular to a central axis (a-a'), mechanical failure as shown in **FIG. 16** is evident.

The plurality of flexibility slots (252) in the distal compliant section (244) may be configured to provide a gradual change, in a longitudinal direction, of flexibility. Preferably, the plurality of flexibility slots (252) in the distal compliant section (244) are arranged such that the distal compliant section (244) is more flexible towards the distal end (40) compared with towards the proximal end (20). The gradual change in flexibility may be achieved, for instance, using flexibility slots (252) of a longer (path) length towards the distal end (40) compared with towards the proximal end (20). The gradual change may be linear.

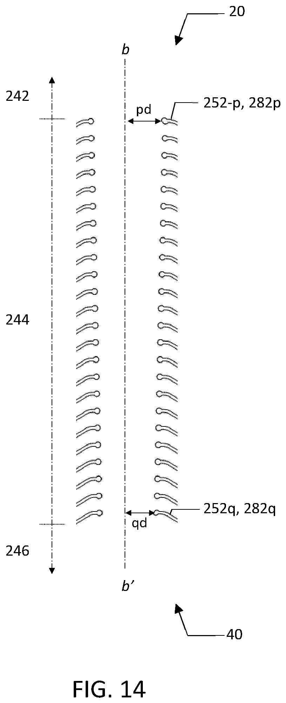

This is exemplified in **FIG. 14****,** showing a net (280) of the wall of the distal compliant section (244), containing several flexibility slots (252-p, 252-q) and represented net slot paths (282-p, 282-q). The flexibility slot (252-p) / net slot path (282-p) located at the proximal end (20) has a shorter length compared with the flexibility slot (252-q) / net slot path (282-q) located at the distal end (40) (pd higher than qd).

When the distal compliant section (244) is more flexible towards the distal end (40) compared with towards the proximal end (20), it has been found that it leads to a improved and more homogenous curvature of the distal compliant section (244) during insertion, compared with when there is no gradual change in flexibility . Without the gradual change in flexibility, the bending is less curved at both the distal and proximal ends of the distal compliant section (244), which can cause tissue damage during insertion.

The distal tip section (246) is an atraumatic wedge-shaped end, configured for entry into the treatment target. In particular, it is configured to prise apart tissue, for instance, the sclera and the choroid (suprachoroidal space), or the conjunctiva and the sclera (subconjunctival space) or within the sclera (intrascleral space). The distal tip section (246) is a part of the delivery shaft (220) in which a transverse profile of the tube wall (224) reduces in size, preferably gradually, in a distal (40) direction. The distal tip section (246) may comprise a flat terminal distal end (advancing edge) (251) of the tube (250) that presents an edge rather than a point to the tissue.

The distal tip section (246) may comprise a gradually narrowed terminal distal end of the tube (250). The gradually narrowed terminal distal end of the tube (250) may be formed by introducing a pair of lateral (56) slots open slots (270) at the distal (40) end of the tube (250) thereby forming a pair of jaws (272, 274), and by compressing the jaws (272, 274) together. Examples of a distal tip section (246) with a gradually narrowed terminal distal end of the tube (250) is illustrated for instance, in **FIGs. 5****,** **10****,** **18, 19** and **20****.**

The tube (250) in the distal tip section (246) may comprise a pair of cuts outs (270-a, 270-b) that define a restricting flap (272) and extension body (274). The restricting flap (272) may be disposed on the anterior (52) side of the tube (250). The extension body (274) may be disposed on the posterior (54) side of the tube (250). The restricting flap (272) and extension body (274) co-operate to form a wedge-shaped distal tip. The wedge advancing edge (251) provides a line of contact with the tissue compared with a needle point. The distal tip section (246) hence provides an atraumatic penetration into the subscleral space.

The restricting flap (272) and extension body (274) may be arranged (diametrically) opposite each other. The restricting flap (272) and extension body (274) may be diametrically arranged around the tube (250).

The cuts outs (270-a, 270-b) extend to the distal terminal end (251) of the tube (250). The cuts outs (270-a, 270-b) are open at the distal end (40). The cuts outs (270-a, 270-b) may be opposite each other. The cuts outs (270-a, 270-b) may be diametrically arranged around the tube (250). Both cuts outs (270-a, 270-b) may be disposed on the lateral sides (56) of the tube (250). A closed end (253) of cuts out (270-a, 270-b) may be in the distal compliant section (244) or in the distal tip section (246), preferably in the distal tip section (246).

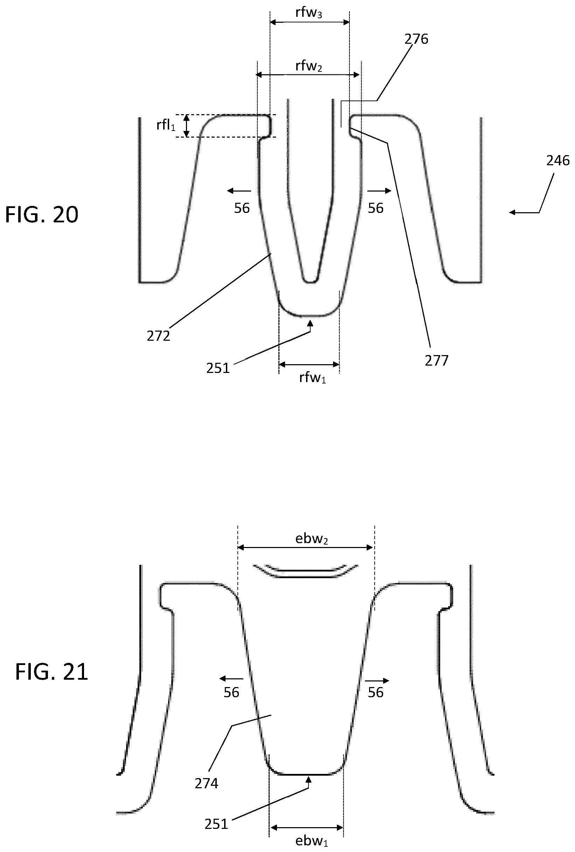

The restricting flap (272) may entirely or for the most part have flat anterior (52) and posterior (54) surfaces. An exemplary restricting flap (272) is shown in detail in **FIG. 20****.** The lateral (56) edges towards a proximal part of the restricting flap (272) may contain a longitudinally curve in a posterior direction. The lateral (56) edges towards a distal part of the restricting flap (272) may be flat. The advancing edge (251) of the distal tip section (246) presents a lateral (56) edge for prising apart tissue.

The proximal end of the restricting flap (272) may have contain a region having a constant width in a distal direction. The width (rfw₂) of the constant width region may be 0.8 mm to 1 mm. The distal end of the restricting flap (272) preferably narrows gradually in a distal direction towards a blunt advancing edge (251). The blunt advancing edge (251) of the restricting flap (272) may have a width (rfw₁) of 0.3 mm to 0.65 mm. The restricting flap (272) may have a bullet-like profile.

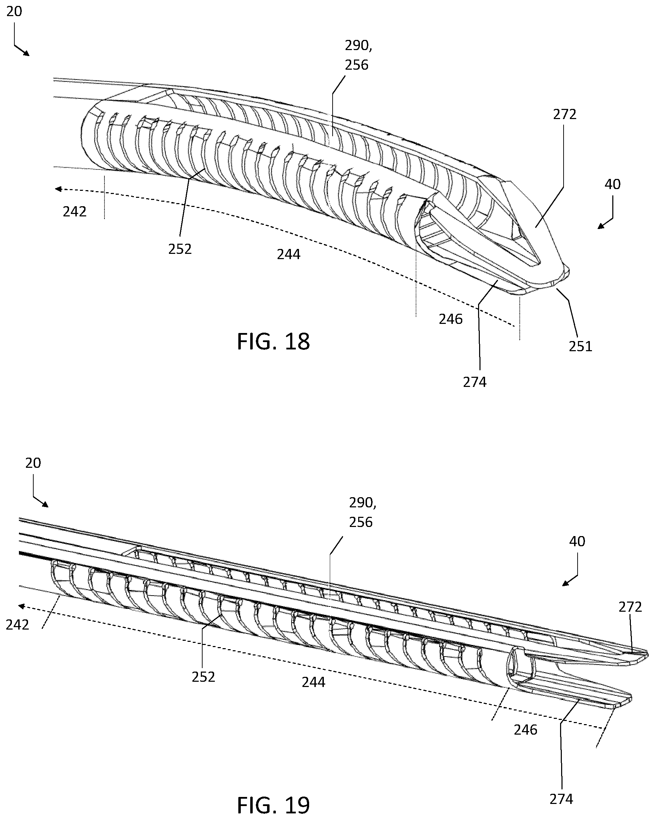

The restricting flap (272) may be moveable between an open state and an occluding state. In an open state, the implant (230) is able to pass through the lumen (222) and out from the delivery shaft for deployment. In the occluding state, passage of the implant (230) through the lumen (222) is blocked. An exemplary distal tip section (246) having a restricting flap (272) in the occluded state is shown in **FIG. 18****.** An exemplary distal tip section (246) having a restricting flap (272) in the open state is shown in **FIG. 19****.** The restricting flap (272) is biased in the occluding state. The restricting flap (272) may be moved from the occluding state to the open state by application of an axial force from the implant (230). The force, in a distal direction, causes the implant to push the restricting flap (272) open from within the lumen (222). In the occluding state, the restricting flap (272) is angled in an posterior (54) direction, they at least partly occluding exit from the lumen. In the occluding state, the restricting flap (272) is angled towards a central axis (a-a') of the tube (250).

The restricting flap (272) may join to the distal end of the distal compliant section (244) with a living hinge (276). A living hinge is exemplified in **FIG. 20****.** The living hinge is preferably a narrowed neck (277) part of the tube (250). The narrowed neck (277) has (lateral) width (rfw₃) that is smaller than of the restricting flap (272) (rfw₂) where it joins the narrowed neck (277) (rfl₁). The (lateral) width across the narrowed neck (277) (rfw₃) may be 0.5 to 0.8 mm. The width (rfw₂) of the restricting flap (272) where it joins the narrowed neck (277) may be 0.8 to 1mm. An axial length (rfl₁) of the narrowed neck (277) may be 0.1 to 0.3 mm.

The observation fenestration (290) may extend into the restricting flap (272) to allow visibility of the implant during deployment. The part of the observation fenestration (290) in the restricting flap (272) may have v- or u-shaped. The part of the observation fenestration (290) in the restricting flap (272) may have an outline that follow the outline shape of the restricting flap...

The extension body (274) may entirely or for the most part have flat anterior (52) and posterior (54) surfaces. An exemplary extension body (274) is shown in detail in **FIG. 21****.** The lateral (56) edges towards a proximal part of the extension body (274) may contain a longitudinally curve (upwards) in an anterior direction. The lateral (56) edges towards a distal part of the extension body (274) may be flat. The extension body (274) preferably narrows gradually in a distal direction towards a blunt advancing edge (251). The proximal end of the extension body (274) may or may not contain a part having a constant width in a distal direction. The extension body (274) may have only one state which is an open state. A longitudinal axis of the extension body (274) may be parallel to a central longitudinal axis (a-a') of the tube. The extension body (274) may have a bullet-like profile. The proximal end of the extension body (274) may have a (lateral) width (ebw₂) of 0.9 mm to 1.3 mm. The advancing edge (251) of the extension body (274) may have a (lateral) width (ebw₁) of 0.5 to 0.85 mm. The advancing edge (251) of the extension body (274) may have rounded lateral edges to reduce tissue trauma.

The restricting flap (272) and extension body (274) advantageously provide a blunt-ended distal tip section (246) that forms a wedge for mechanically prising apart the treatment tissue upon implant deployment. The pair of surfaces (anterior and posterior) provided by the restricting flap (272) and extension body (274) support the treatment tissue with a larger surface area compared with a conventional needle tip, thereby reducing trauma and bleeding. Furthermore, the closed restricting flap (272) retains the implant within the lumen, preventing loss of the implant during storage, transport and handling.

As described earlier, at least a part of the delivery shaft (240) may be provided with an axially-longitudinal observation fenestration (290) (see **FIGs. 2A****,** **4C****,** **5****,** **10****,** **11** to **13A, 18, 19).** The observation fenestration (290) comprises a longitudinal aperture (256) in the tube wall (224) on the anterior (52) side, which fenestration connects the lumen (222) to the exterior of the tube (250). The observation fenestration (290) allows visualisation of the implant (230), in particular of the marker (205). It allows the implant (230) to be visualised relative to the treatment target prior to deployment. It allows the deployment of implant (230) to visualised. The observation fenestration (290) may be disposed on an anterior (52) side of the tube (250). The observation fenestration (290) may be limited to an anterior (52) side of the tube (250). The inner edge (292) of the aperture (256) forms a closed path.

The observation fenestration (290) may be disposed at least partly in the distal compliant section (244). The observation fenestration (290) preferably extends from the distal compliant section (244) into the distal tip section (246). The observation fenestration (290) may or may not extend from the distal compliant section (244) into a part of the proximal section (242).

The observation fenestration (290) may be defined by a continuous inner edge (292). The continuous inner edge (292) is illustrated, for instance, in **FIG. 11****.** By continuous inner edge, it is meant that the edge is not breached by one or more flexibility slots that would interrupt the edge continuity. The edge is space between an inner and outer surface of the tube (250). Longitudinal inner edges (292a, 292b) of the observation fenestration (290) may in particular be continuous. Longitudinal inner edges (292a, 292b) of the observation fenestration (290) may in particular be straight. The inventors have found that the continuous inner edge (292) of the observation fenestration (290) prevented damage to the compliant section (244) when the delivery shaft (220) is withdrawn over the discharge shaft (510) or when the discharge shaft (510) is advanced into the delivery shaft (220). Because the distal compliant section (244) is biased in a curve, and the discharge shaft (510) is straighter, uncurving forces are applied by the discharge shaft (510) to the anterior side (52) of the distal compliant section (244). It has been found that the uncurving force causes failure of distal compliant section (244) when the inner edge (292) of the observation fenestration (290) is not continuous (see **FIG. 15A** and **B****).**

The observation fenestration (290) may be defined by or surrounded by a border (294) that is a region of the delivery shaft (220) tube (250) wall around the observation fenestration (290). The wall means the inner and outer surface of the tube (250) and the thickness of tube material between them. The border (294) is illustrated, for instance, in **FIGs. 12, 13** and **13A****.** The border is continuous or intact, namely it is not breached by one or more flexibility slots. In an example of **FIG. 13A****,** the flexibility slots (252) rounded apertures (254) abut longitudinal outer edges of the border in the distal compliant section (244).

The border has opposing long sides (294a, 294b). Each border long side (294a, 294b) adjoins the longitudinal inner edges (292a, 292b) of the observation fenestration (290). The border long sides (294a, 294b) are joined at either end by shorter sides. The border proximal short side is preferably straight. The border distal short side is preferably formed from the restricting flap (272).

Each border long side (294a, 294b) may have a width (bw) dimension. The width of each border long side (294a, 294b) may, in the distal compliant section (244), be **constant** in a longitudinal direction; this is shown, for instance, in **FIG. 12****.** The width of each border long side (294a, 294b) may **gradually,** in the distal compliant section (244), decrease towards the distal end of the distal compliant section (244); this is shown, for instance, in **FIG. 13****.**

Each border long side (294a, 294b) may have a width (bw), in the distal compliant section (244), having a minimum value in the range 0.2 mm to 0.5 mm, preferably 0.25 to 0.40. Where width of each border long side (294a, 294b), in the distal compliant section (244), gradually decreases towards the distal end of the distal compliant section (244), the largest width (bw₁) in the distal compliant section (244) may be a value in the range 0.3 mm to 0.4 mm and the smallest width (bw₂) in the distal compliant section (244) may be a value in the range 0.2 mm to 0.3 mm.

Having a border reduces the incidence of structural failure of the distal compliant section during implant deployment. It was observed that when the implant was released with a certain angle and speed the discharge shaft (510), the flexibility slot (252) opening into the observation fenestration (290) would mechanically fail, and the discharge shaft (510) would go through the observation fenestration (290) **(****FIGs. 15A** and **B****).** This was considered as an unacceptable risk. The problem of mechanical failure was solved by the presence of the continuous (uninterrupted) border (294).

Having a border width that gradually decrease towards the distal end gradually increases flexibility of the distal compliant section (244) at the distal end compared with the proximal end.

When the distal compliant section (244) is more flexible towards the distal end (40) compared with towards the proximal end (20), it has been found that it leads to a improved and more homogenous curvature of the distal compliant section (244) during insertion, compared with when there is no gradual change in flexibility. Without the gradual change in flexibility, the bending is less curved at both the distal and proximal ends of the distal compliant section (244), which can cause tissue damage during insertion.

The observation fenestration (290) may span a portion of the transverse cross-section perimeter path (62) of the tube (250). The observation fenestration (290) width (*ow*) **(****FIGs. 2C****,** **4C****,** **11****)** may be equal to or greater than 0.3 mm. It may be less than 0.5 mm. A size between 0.3 and 0.5 mm ensures good visibility of the implant. The observation fenestration (290) width (ow) is indicated in **FIGs. 2C****,** **4C** and **11****.** An axial or longitudinal length (ol) of the observation fenestration (290) may be equal to or greater than 6 mm. It may be less than 20 mm. Preferably the axial or longitudinal length is between 6 and 7 mm. The axial or longitudinal length (ol) may be greater than that of the implant to ensure full visualization of the implant and of the discharge shaft (510). The observation fenestration (290) may be dimensioned to retain the implant (230) prior to and during deployment.

The delivery shaft assembly (200) further comprises an adapter (210) configured for coupling to an inserter tool (500). The implant (230) is disposed at or towards a distal end (40) of the delivery shaft (220). The adapter (210) is disposed at a proximal end (20) of the delivery shaft (220). The adapter (210) has a body provided with a receiving space (212) for a part of the inserter tool (500) and an aperture (214) connecting the receiving space (212) to a lumen (222) of delivery shaft (220) (see **FIG. 2****).** The lumen (222) is configured for the passage of a discharge shaft (510) of the inserter tool (500) therethrough. The implant (230) may be ejected from the delivery shaft (220) by actuation of the inserter tool (500), thereby delivering the implant (230) to the treatment target. An exemplary delivery shaft assembly (200) is disclosed in WO 2017/108498, which is incorporated herein by reference.

The proximal section (242) is adjacent to the distal compliant section (244) and extends in a proximal direction to the adapter (210). The proximal section (242) may be straight across its entire axial length. The proximal section (242) of the tube (250) may be bend-resistive across its full axial length. The bend-resistiveness is an inherent property of the bend-resistive tube (250). Examples where the tube (250) in the proximal section is bend-resistive across its full axial length is shown in **FIGs. 5****,** **6, 7,** and **8****.**

The proximal section (242) may contain one or more compliant portions. The one or more compliant portions may be biased in a straight line. The one or more compliant portions may bend in the is at least in the 1^{st} plane, for instance, in a range of planes including or centred on the 1^{st} plane, or is limited to the 1^{st} plane.

Where one or more compliant portions is present in the proximal section (242), the compliance and bendability is achieved by a plurality of flexibility slots (252). A flexibility slot is a discrete transverse or lateral (56) slot (252) in the wall (224) of the tube (250). It extends partially around a perimeter (62) of the tube wall (224). A transverse length of the slot (*sl*) is greater than its width. The slot path is preferably straight. A majority or all of the flexibility slots (252) in a compliant portion may be closed *i.e.* the internal edges of the slot form a continuous path.

A detail of a flexibility slot (252) is show in **FIG. 4A****.** The path of a flexibility slot (252) may coincide with a plane parallel to a central axis (a-a') of the tube (250). The flexibility slot comprise a rounded aperture (254) at one or both of its terminal ends (see **FIG. 4B****).** The rounded aperture (254) may have a circular form.

Each flexibility slot (252) may span a portion of the transverse cross-section perimeter path (62) of the tube (250). The transverse cross-section perimeter path (also known a perimeter path) (62) of the tube (250) refers to the outer path of the tube along a transverse cross-section (see **FIG. 2D****).** The flexibility slot length (*sl*) in the compliant portion of the proximal section (242) may be a portion of the length (*pl*) of the perimeter path (62) of the tube (250), for instance, 1 % to 80 %, for instance 1 % to 40%.

A flexibility slot (252) in the compliant portion may be positioned only within a posterior (54) half or only within an anterior (52) half of the transverse cross-section. A majority of or all flexibility slot (252) in the compliant portion is spaced apart in an axial direction. The flexibility slots are preferably arranged in one or more (preferably two) rows extending in a proximal to distal direction.

A majority of or all the flexibility slots (252) in the compliant portion may be arranged such that bendability is at least in the 1^{st} plane, for instance, in a range of planes including or centred on the 1^{st} plane, or is limited to the 1^{st} plane.

At least a part of the proximal section (242) may be provided with an observation fenestration (290), as described above. The observation fenestration (290) may be limited to and disposed an anterior (52) side of the tube (250). The observation fenestration (290) may be a closed aperture (256) *i.e.* the edges of the aperture form a closed path. The observation fenestration (290) may extend distally (40) into the distal compliant section (244).

The proximal section (242) may have a length in an axial (a-a') direction of 26 to 30 mm. The length is measured along a central axis (a-a') from the distal end of the adaptor (210) to the proximal end of the distal compliant section (244). The proximal section (242) may have a maximum outer tube width across the lateral sides (*tw* in **FIG. 2A****)** of 0.9 mm to 1.5 mm, preferably about 1.05 mm to 1.25 mm, more preferably 1.15±0.1 mm. The delivery shaft (240) may have an outer tube height across the posterior-anterior side of (*th* in **FIG. 2A****)** of 0.5 mm to 0.7 mm, preferably about 0.52 mm to 1.24 mm.

The delivery shaft (220) or tube (250) may be polished. The polished delivery shaft (220) or tube (250) may be achieved by mechanical polishing, chemical polishing (etching), or a combination of mechanical and chemical polishing. The polishing has several effects:
- produces smoother curves and rounded edges that prevents the formation of cracks,
- rounds edges to avoid tissue trauma
- removes burrs to avoid tissue trauma
- elimination of oxidated surface of the Nitinol after shaping
- Chemical polishing gives a light reflective finish to the lumen (222). Because the hydrated implant (230) has a transparent or translucent, light passing through the implant reflect back towards the user, and thereby increasing visual contrast between the implant and one or more markers (205).

Chemical polishing may be achieved by treatment in an acid bath.

At least the delivery shaft (220) and optionally the adaptor (210) may be disposed with a sheath to protect at least the delivery shaft (220) and optionally the adaptor (210). The sheath may be made from a heat-shrink polymer such as polyester heat shrink film.

Provided herein is a method for manufacturing the delivery shaft assembly as described herein. The delivery shaft (220) and the portions and sections are preferably formed from one piece of tube (250). The flexibility slots (252), observation fenestration (290), cut-outs (270-a, 270-b), and/or living hinge (276), are introduced into the tube (250) any method any method for removal of tube material from the tube wall (224). Exemplary methods include laser cutting, photochemical etching, deep pressing, conventional chipping techniques such as drilling or milling, high pressure water jet cutting systems or any suitable material removing process available. Preferably laser cutting is used as this allows a very accurate and clean removal of material under reasonable economic conditions.

A method for manufacturing the delivery shaft assembly as described herein comprises one or more, preferably all, of the following steps:
- introducing the plurality of flexibility slots (252) in the distal compliant section (244) to impart the compliance and bendability by removal of tube material from the tube wall (224);
- introducing the observation fenestration (290) by removal of tube material from the tube wall (224);
- introducing other features where present including the flexibility slots (252) in the proximal section (242), cut-outs (270-a, 270-b), and/or living hinge (276) by removal of tube material from the tube wall (224);
- introducing the oblong transverse profile of the delivery shaft (220) tube (250). This may be introduced by any number of ways, for instance, by inserting an oblong transverse profiled mandrel into the lumen (222) of the cylindrical tube (150) and applying radial pressure at high temperature (e.g. 500 deg C);
- introducing the curve of the distal compliant section (244). This may be introduced by any number of ways, for instance, moulding the curve at a high temperature (e.g. 500 deg C);
- introducing the biasing of the restrictive flap (272) into the occluding state. This may be introduced by any number of ways, for instance, by moulding the restrictive flap (272) into the occluding state at high temperature (e.g. 500 deg C);
- polishing the cut tube. This may be done by any number of ways for instance mechanically and/or chemically (etching);
- attachment to the adaptor (210). This may be done by any number of ways for instance using an adhesive.

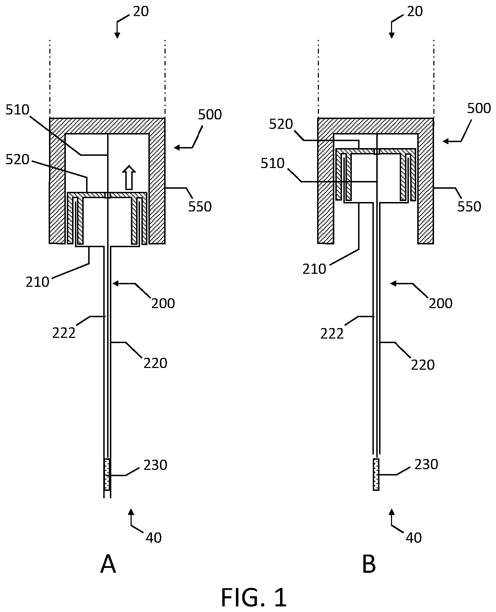

The inserter tool (500) is provided with a discharge shaft (510) configured to be received by the delivery shaft assembly lumen (222). The discharge shaft (510) may be configured to abut the implant (230). Movement of the delivery shaft (220) and/or of the discharge shaft (510) causes release of the implant (230) from the lumen (222) (see **FIG. 1****, Panels A** and **B).** The discharge shaft (510) is preferably straight. The discharge shaft (510) causes the distal compliant section (244), biased in a curve, to uncurve.

Advancement of the discharge shaft (510) in a distal (40) direction may apply force to the implant (230), causing it to be ejected from the shaft assembly lumen (220). Alternatively or additionally, withdrawal of the delivery shaft (220) relative to a fixed discharge shaft (510) causes the implant (230) to be ejected from the shaft assembly lumen (220).

The inserter tool (500) is provided with an adapter coupling (520) configured to couple with the adapter (210). The coupling between the adapter coupling (520) and adapter (210) may be releasable or non-releasable. A non-releasable coupling may be achieved, for instance, by providing a compliant member on one of the adapter coupling (520) or adapter (210) and a reciprocating stop member on the other, wherein the compliant member slides across the stop member in one direction during couple the parts (520, 210) and the compliant member engages with the stop member in the other sliding direction to prevent release of the parts (520, 210). The mechanism is similar to the one-directional movement of a ratchet mechanism.

Where the implant is ejected by withdrawal of the delivery shaft (220) relative to the fixed discharge shaft (510), the adapter coupling (520) may be slidable relative to the fixed discharge shaft (510). The slidable adapter coupling (520) has a primed and deployed position. In the primed position, the slidable adapter coupling (520) places the adapter (210) and delivery shaft lumen (222) in a distal-most position. The fixed discharge shaft (510) may abut the implant (230) and the delivery shaft lumen (222) may cover the implant (230). In the deployed position, the slidable adapter coupling (520) places the adapter (210) and delivery shaft lumen (222) in a proximal-most position. The discharge shaft (510) abuts the implant (230) and the delivery shaft lumen (222) is withdrawn in a proximal (20) direction and the implant (230) is released. Where the implant is ejected by advancement of the discharge shaft (510), the discharge shaft (510) slidable relative to a fixed adapter coupling (520) applied an ejection force in a distal direction upon the implant (230) causing it to be ejected from the lumen (222).

An example of a part of an inserter tool (500) is provided in **FIG. 1****,** showing an inserter housing or chassis (550) (fixed), and a slidable adapter coupling (520) configured to engage with the adapter (210) of the delivery shaft assembly (200). A discharge shaft (510) is disposed in fixed relation to the housing (550). In this example, the adapter coupling (520) is slidable relative to the housing (550) and is shown in a primed position; it is appreciated that the other configurations of the inserter tool (500) exist, for instance, in which the adapter coupling (520) is disposed in fixed relation to an inserter tool housing and the discharge shaft (510) is slidable. In **Panel A,** the inserter tool (500) is shown in the primed position; the adapter coupling (520) is in a distal (40) position. In the primed position, the implant (230) is retained in the delivery shaft lumen (222). In **Panel B,** the inserter tool (500) is shown in the deployed position; the adapter coupling (520) is withdrawn to a proximal (20) position. In the deployed position, the delivery shaft is withdrawn, thereby releasing the implant (230) from the delivery shaft lumen (222). An exemplary inserter tool (500) is disclosed in WO 2017/108498.

The implantation of implant 230 may proceed using, for instance, an *ab interno* method. The implant (230) is retained within the delivery shaft lumen (222) of the delivery shaft (220) which is mounted on the discharge shaft (510) of the inserter tool (500). The distal tip section (246) may be used to ease penetration of the delivery shaft (220) through the cornea. It will readily be appreciated that the distal tip section (246) does not need to provide the incision into the cornea and this can be done by a separate tool with the delivery shaft (220) being inserted into the incision made. The delivery shaft (220) is directed into and across the anterior chamber of the eye to the iridocorneal angle and into the sub-scleral space. The distal tip section (246) provides an atraumatic penetration into the sub-scleral space. The delivery shaft (220) is retracted with respect to the discharge shaft (510) of the inserter tool (500) leaving implant (230) in position within the subscleral space.

The inserter tool (500) may be an implantation device as described in WO 2017/108498. The delivery shaft assembly (200) may comprise a one-touch fitting connection element or adaptor (210) as described in WO 2017/108498. For example, WO 2017/108498 describes an implantation device (500) and delivery shaft assembly (200) on pages 26 to page 33.

## Claims

1. A delivery shaft assembly (200) having a proximal end (20) and a distal end (40) for delivery of an ocular implant (230), wherein:
- delivery shaft assembly (200) comprises a delivery shaft (220) having a lumen (222) configured for holding the implant (230), and an adapter (210) at the proximal end (20) of the delivery shaft (220) configured for attachment to an inserter tool (500) for deployment of the implant (230);
- the delivery shaft (220) comprises a distal compliant section (244) that is repeatably bendable, compliant, and biased in a curve in a 1^{st} plane (60);
- the delivery shaft (220) is provided with an axially longitudinal observation fenestration (290) at least partly disposed in the distal compliant section (244) allowing visualisation of the implant (230) from an anterior (52) side of the delivery shaft (220);
- the delivery shaft (220) comprises a distal tip section (246) having an atraumatic distal tip, **characterized in that**
- a wall (224) of the tube (250) in the distal compliant section (244) is provided with a plurality of flexibility slots (252) that impart the bendability,
- the wall (224) of the tube (250) in the distal tip section (246) comprises a pair of cut outs (270-a, 270-b) that define a restricting flap (272) and an extension body (274), wherein, the restricting flap (272) and extension body (274) co-operate to form a wedge-shaped distal tip, and the restricting flap (272) is moveable around a living hinge (276) between an open state and an occluding state and is biased in the occluding state.

2. The delivery shaft assembly (200) according to claim 1, wherein the observation fenestration (290) is defined by an inner edge (292) that is continuous.

3. The delivery shaft assembly (200) according to claim 1 or 2, wherein the observation fenestration (290) is surrounded by a border (294) that is a region of the delivery shaft (220) tube (250) wall around the observation fenestration (290), wherein the border is continuous.

4. The delivery shaft assembly (200) according to claim 3, wherein the border (294) has two opposing long sides (294-a, 294-b), and each border long side (294-a, 294-b) in the distal compliant section (244) gradually decreases in width towards the distal end (40) of the distal compliant section (244).

5. The delivery shaft assembly (200) according to any one of claims 1 to 4, wherein the observation fenestration (290) is continuous and spans at least a part of the distal compliant section (244) and of the distal tip section (246).

6. The delivery shaft assembly (200) according to any one of claims 1 to 5, wherein the plurality of flexibility slots (252) are spaced apart in at least a longitudinal direction.

7. The delivery shaft assembly (200) according to any one of claims 1 to 6, wherein each flexibility slot (252-a; 252-b) of the plurality of flexibility slots (252) has a net slot path (282) containing one or more inclined portions (284), and a pair of longitudinally adjacent flexibility slots (252-a; 252-b) each contain one or more inclined portions (284-a; 284-b), and a plane perpendicular to a central longitudinal axis (a-a') of the tube (250) crosses both longitudinally adjacent flexibility slots (252-a; 252-b).

8. The delivery shaft assembly (200) according to any one of claims 1 to 7, wherein a radial perimetric integrity is equal to or greater than 50% at all positions along the longitudinal length of the distal compliant section (244), wherein the radial perimetric integrity is a proportion of a perimeter length of wall material present in a plane perpendicular to the central axis (a-a') of the tube (250) in the distal compliant section (244), compared with a length of the perimeter (*pl*) of the intact tube (250) in the same plane.

9. The delivery shaft assembly (200) according to claim 7 or 8, wherein the net slot path (282) of each flexibility slot (252) in the distal compliant section (244) contains two inclined portions (284-a, 284-b) connected on the posterior side (54) of the tube (250), preferably by a perpendicular portion (286), and each flexibility slot (252) of the plurality has an identical net slot path (282).

10. The delivery shaft assembly (200) according to claim 9, wherein each inclined portion (284-a, 284-b) extends towards the anterior side (52) of the delivery shaft (220) into a perpendicular portion (284-a, 284-b), and optionally each anterior side (52) perpendicular portion (284-a, 284-b) terminates in a rounded aperture (254-a, 254-b).

11. The delivery shaft assembly (200) according to claim 9 or 10, wherein an angle, alpha, of an incline of each inclined portion (284-a, 284-b) is the same and is 55 to 65 deg.

12. The delivery shaft assembly (200) according to any one of claims 9 to 11 wherein, a distance (sd) between adjacent flexibility slots (252) is the same for the flexibility slots (252) of plurality, and is a value in a range 0.20 to 0.30 mm.

13. The delivery shaft assembly (200) according to claim 7 or 8, wherein
- the plurality of flexibility slots (252) in the distal compliant section (244) is arranged into a set of narrower flexibility slots (252-e₁, 282-e₁, 252-e₂, 282-e₂) and a set of wider flexibility slots (252-f, 282-f)
- the narrower (252-e₁, 282-e₁, 252-e₂, 282-e₂) and wider (252-f, 282-f) flexibility slots alternate and are spaced apart in longitudinal direction,
- each wider flexibility slots 252-f, 282-f) contain one continuous inclined portion (284-f), and
- each narrower flexibility slot (252-e₁, 282-e₁, 252-e₂, 282-e₂) contain two continuous inclined portions (284-e₁, 284-e₂), spaced apart by an intact section (283-e) of tube (250) wall on the posterior side (54) of the tube (250).

14. The delivery shaft assembly (200) according to claim 13, wherein an angle, alpha, of an incline of each inclined portion (284-e₁, 284-e₂, 284-f) is the same and is 55 to 65 deg.

15. The delivery shaft assembly (200) according to any one of claims 1 to 14, wherein the curve of the distal compliant section (244) contains a radius of between 30 to 50 mm.

## Patentansprüche

1. Zuführschaftbaugruppe (200) mit einem proximalen Ende (20) und einem distalen Ende (40) zum Zuführen eines Augenimplantats (230), wobei:
- die Zuführschaftbaugruppe (200) einen Zuführschaft (220) mit einem Lumen (222), das zum Halten des Implantats (230) ausgestaltet ist, und einen Adapter (210) an dem proximalen Ende (20) des Zuführschafts (220) umfasst, der zum Anbringen an einem Einführwerkzeug (500) zum Einsetzen des Implantats (230) ausgestaltet ist,
- der Zuführschaft (220) einen distalen nachgiebigen Abschnitt (244) umfasst, der wiederholt biegbar, nachgiebig und in einer Krümmung in einer 1. Ebene (60) vorgespannt ist,
- der Zuführschaft (220) mit einer axial länglichen Beobachtungsfensterung (290) versehen ist, die mindestens teilweise in dem distalen nachgiebigen Abschnitt (244) angeordnet ist und eine Visualisierung des Implantats (230) von einer vorderen (52) Seite des Zuführschafts (220) aus gestattet,
- der Zuführschaft (220) einen distalen Spitzenabschnitt (246) mit einer atraumatischen distalen Spitze umfasst, **dadurch gekennzeichnet, dass**
- eine Wand (224) des Rohrs (250) in dem distalen nachgiebigen Abschnitt (244) mit einer Vielzahl von Flexibilitätsschlitzen (252) versehen ist, die die Biegbarkeit verleihen,
- die Wand (224) des Rohrs (250) in dem distalen Spitzenabschnitt (246) ein Paar Ausschnitte (270-a, 270-b) umfasst, die eine Begrenzungsklappe (272) und einen Verlängerungskörper (274) definieren, wobei die Begrenzungsklappe (272) und der Verlängerungskörper (274) zusammenwirken, um eine keilförmige distale Spitze zu bilden, und die Begrenzungsklappe (272) um ein Filmscharnier (276) herum zwischen einem offenen und einem okkludierenden Zustand beweglich und in dem okkludierenden Zustand vorgespannt ist.

2. Zuführschaftbaugruppe (200) nach Anspruch 1, wobei die Beobachtungsfensterung (290) durch eine inneren Rand (292) definiert ist, die durchgehend ist.

3. Zuführschaftbaugruppe (200) nach Anspruch 1 oder 2, wobei die Beobachtungsfensterung (290) von einer Umrandung (294) umgeben ist, die ein Bereich der Wand des Rohrs (250) des Zuführschafts (220) um die Beobachtungsfensterung (290) herum ist, wobei die Umrandung durchgehend ist.

4. Zuführschaftbaugruppe (200) nach Anspruch 3, wobei die Umrandung (294) zwei gegenüberliegende lange Seiten (294-a, 294-b) aufweist und die Breite jeder langen Seite (294-a, 294-b) der Umrandung in dem distalen nachgiebigen Abschnitt (244) allmählich zu dem distalen Ende (40) des distalen nachgiebigen Abschnitts (244) hin abnimmt.

5. Zuführschaftbaugruppe (200) nach einem der Ansprüche 1 bis 4, wobei die Beobachtungsfensterung (290) durchgehend ist und mindestens einen Teil des distalen nachgiebigen Abschnitts (244) und des distalen Spitzenabschnitts (246) überspannt.

6. Zuführschaftbaugruppe (200) nach einem der Ansprüche 1 bis 5, wobei die Vielzahl von Flexibilitätsschlitzen (252) in mindestens einer Längsrichtung beabstandet sind.

7. Zuführschaftbaugruppe (200) nach einem der Ansprüche 1 bis 6, wobei jeder Flexibilitätsschlitz (252-a; 252-b) der Vielzahl von Flexibilitätsschlitzen (252) einen Netz-Schlitzpfad (282) aufweist, der einen oder mehrere geneigte Abschnitte (284) enthält, und ein Paar in Längsrichtung benachbarter Flexibilitätsschlitze (252-a; 252-b) jeweils einen oder mehrere geneigte Abschnitte (284-a; 284-b) enthalten und eine senkrecht zu einer mittleren Längsachse (a-a') des Rohrs (250) verlaufende Ebene beide in Längsrichtung benachbarte Flexibilitätsschlitze (252-a; 252-b) kreuzt.

8. Zuführschaftbaugruppe (200) nach einem der Ansprüche 1 bis 7, wobei eine radiale Umfangsintegrität an allen Positionen entlang der Längslänge des distalen nachgiebigen Abschnitts (244) gleich oder größer als 50% ist, wobei die radiale Umfangsintegrität ein Anteil einer Umfangslänge von Wandmaterial ist, das in einer senkrecht zu der Mittelachse (a-a') des Rohrs (250) in dem distalen nachgiebigen Abschnitt (244) verlaufenden Ebene im Vergleich zu einer Länge des Umfangs (pl) des intakten Rohrs (250) in der gleichen Ebene vorhanden ist.

9. Zuführschaftbaugruppe (200) nach Anspruch 7 oder 8, wobei der Netz-Schlitzpfad (282) jedes Flexibilitätsschlitzes (252) in dem distalen nachgiebigen Abschnitt (244) zwei geneigte Abschnitte (284-a, 284-b) enthält, die an der hinteren Seite (54) des Rohrs (250) vorzugsweise durch einen senkrechten Abschnitt (286) verbunden sind, und jeder Flexibilitätsschlitz (252) der Vielzahl einen identischen Netz-Schlitzpfad (282) aufweist.

10. Zuführschaftbaugruppe (200) nach Anspruch 9, wobei sich jeder geneigte Abschnitt (284-a, 284-b) zu der vorderen Seite (52) des Zuführschafts (220) in einen senkrechten Abschnitt (284-a, 284-b) hin erstreckt und optional jeder senkrechte Abschnitt (284-a, 284-b) der vorderen Seite (52) in einer abgerundeten Öffnung (254-a, 254-b) endet.

11. Zuführschaftbaugruppe (200) nach Anspruch 9 oder 10, wobei ein Winkel alpha einer Neigung jedes geneigten Abschnitts (284-a, 284-b) gleich ist und 55 bis 65 Grad beträgt.

12. Zuführschaftbaugruppe (200) nach einem der Ansprüche 9 bis 11, wobei ein Abstand (sd) zwischen benachbarten Flexibilitätsschlitzen (252) für die Flexibilitätsschlitze (252) der Vielzahl gleich ist und ein Wert in einem Bereich von 0,20 bis 0,30 mm ist.

13. Zuführschaftbaugruppe (200) nach Anspruch 7 oder 8, wobei
- die Vielzahl von Flexibilitätsschlitzen (252) in dem distalen nachgiebigen Abschnitt (244) in einem Satz schmalerer Flexibilitätsschlitze (252-e₁, 282-e₁, 252-e₂, 282-e₂) und einem Satz breiterer Flexibilitätsschlitze (252-f, 282-f) angeordnet sind,
- sich die schmaleren (252-e₁, 282-e₁, 252-e₂, 282-e₂) und breiteren (252-f, 282-f) Flexibilitätsschlitze abwechseln und in Längsrichtung voneinander beabstandet sind,
- jeder breitere Flexibilitätsschlitz (252-f, 282-f) einen durchgehenden geneigten Abschnitt (284-f) enthält und
- jeder schmalere Flexibilitätsschlitz (252-e₁, 282-e₁, 252-e₂, 282-e₂) zwei durchgehende geneigte Abschnitte (284-e₁, 284-e₂) enthält, die durch einen intakten Abschnitt (283-e) der Wand des Rohrs (250) an der hinteren Seite (54) des Rohrs (250) beabstandet sind.

14. Zuführschaftbaugruppe (200) nach Anspruch 13, wobei ein Winkel alpha einer Neigung jedes geneigten Abschnitts (284-e₁, 284-e₂, 284-f) gleich ist und 55 bis 65 Grad beträgt.

15. Zuführschaftbaugruppe (200) nach einem der Ansprüche 1 bis 14, wobei die Krümmung des distalen nachgiebigen Abschnitts (244) einen Radius zwischen 30 und 50 mm enthält.

## Revendications

1. Ensemble formant tige de pose (200) comportant une extrémité proximale (20) et une extrémité distale (40) pour la pose d'un implant oculaire (230), où :
- l'ensemble formant tige de pose (200) comprend une tige de pose (220) comportant une lumière (222) conçue pour maintenir l'implant (230), et un adaptateur (210) au niveau de l'extrémité proximale (20) de la tige de pose (220) conçu pour être fixé à un outil d'insertion (500) pour la mise en place de l'implant (230) ;
- la tige de pose (220) comprend une section distale flexible (244) qui est déformable de manière répétée, flexible et sollicitée de façon à définir une courbe dans un 1^{er} plan (60) ;
- la tige de pose (220) est pourvue d'une ouverture d'observation axialement longitudinale (290) disposée au moins partiellement dans la section distale flexible (244) permettant de voir l'implant (230) depuis un côté antérieur (52) de la tige de pose (220) ;
- la tige de pose (220) comprend une section de pointe distale (246) comportant une pointe distale atraumatique, **caractérisé en ce que**
- une paroi (224) du tube (250) dans la section distale flexible (244) est pourvue d'une pluralité de fentes de flexibilité (252) qui confèrent la déformabilité,
- la paroi (224) du tube (250) dans la section de pointe distale (246) comprend une paire d'échancrures (270-a, 270-b) qui définissent une languette de restriction (272) et un corps de prolongement (274), la languette de restriction (272) et le corps de prolongement (274) coopérant pour former une pointe distale en forme de coin, et la languette de restriction (272) étant mobile autour d'une charnière souple (276) entre un état ouvert et un état obstructif et étant sollicitée de façon à être dans l'état obstructif.

2. Ensemble formant tige de pose (200) selon la revendication 1, dans lequel l'ouverture d'observation (290) est définie par un bord intérieur (292) qui est continu.

3. Ensemble formant tige de pose (200) selon la revendication 1 ou 2, dans lequel l'ouverture d'observation (290) est entourée par une bordure (294) qui est une région de la paroi du tube (250) de la tige de pose (220) autour de l'ouverture d'observation (290), dans lequel la bordure est continue.

4. Ensemble formant tige de pose (200) selon la revendication 3, dans lequel la bordure (294) a deux côtés longs opposés (294-a, 294-b), et chaque côté long de bordure (294-a, 294-b) dans la section distale flexible (244) diminue progressivement en largeur vers l'extrémité distale (40) de la section distale flexible (244).

5. Ensemble formant tige de pose (200) selon l'une quelconque des revendications 1 à 4, dans lequel l'ouverture d'observation (290) est continue et couvre au moins une partie de la section distale flexible (244) et de la section de pointe distale (246).

6. Ensemble formant tige de pose (200) selon l'une quelconque des revendications 1 à 5, dans lequel la pluralité de fentes de flexibilité (252) sont espacées au moins dans une direction longitudinale.

7. Ensemble formant tige de pose (200) selon l'une quelconque des revendications 1 à 6, dans lequel chaque fente de flexibilité (252-a ; 252-b) de la pluralité de fentes de flexibilité (252) a un chemin de fente de patron (282) contenant une ou plusieurs parties inclinées (284), et une paire de fentes de flexibilité (252-a ; 252-b) longitudinalement adjacentes contiennent chacune une ou plusieurs parties inclinées (284-a ; 284-b), et un plan perpendiculaire à un axe longitudinal central (a-a') du tube (250) croise les deux fentes de flexibilité (252-a ; 252-b) longitudinalement adjacentes.

8. Ensemble formant tige de pose (200) selon l'une quelconque des revendications 1 à 7, dans lequel une intégrité périmétrique radiale est égale ou supérieure à 50 % à toutes les positions le long de la longueur longitudinale de la section distale flexible (244), dans lequel l'intégrité périmétrique radiale est une proportion d'une longueur périmétrique de matériau de paroi présent dans un plan perpendiculaire à l'axe central (a-a') du tube (250) dans la section distale flexible (244), comparée à une longueur du périmètre (pl) du tube intact (250) dans le même plan.

9. Ensemble formant tige de pose (200) selon la revendication 7 ou 8, dans lequel le chemin de fente de patron (282) de chaque fente de flexibilité (252) dans la section distale flexible (244) contient deux parties inclinées (284-a, 284-b) reliées sur le côté postérieur (54) du tube (250), de préférence par une partie perpendiculaire (286), et chaque fente de flexibilité (252) de la pluralité a un chemin de fente de patron (282) identique.

10. Ensemble formant tige de pose (200) selon la revendication 9, dans lequel chaque partie inclinée (284-a, 284-b) s'étend vers le côté antérieur (52) de la tige de pose (220) en une partie perpendiculaire (284-a, 284-b), et facultativement chaque partie perpendiculaire (284-a, 284-b) du côté antérieur (52) se termine par une ouverture arrondie (254-a, 254-b).

11. Ensemble formant tige de pose (200) selon la revendication 9 ou 10, dans lequel un angle, alpha, d'une inclinaison de chaque partie inclinée (284-a, 284-b) est le même et est de 55 à 65 degrés.

12. Ensemble formant tige de pose (200) selon l'une quelconque des revendications 9 à 11, dans lequel une distance (sd) entre des fentes de flexibilité (252) adjacentes est la même pour les fentes de flexibilité (252) de la pluralité, et est une valeur dans une plage de 0,20 à 0,30 mm.

13. Ensemble formant tige de pose (200) selon la revendication 7 ou 8, dans lequel
- la pluralité de fentes de flexibilité (252) dans la section distale flexible (244) sont agencées en un ensemble de fentes de flexibilité plus étroites (252-e₁, 282-e₁, 252-e₂, 282-e₂) et un ensemble de fentes de flexibilité plus larges (252-f, 282-f)
- les fentes de flexibilité plus étroites (252-e₁, 282-e₁, 252-e₂, 282-e₂) et plus larges (252-f, 282-f) sont alternées et espacées dans la direction longitudinale,
- chaque fente de flexibilité plus large (252-f, 282-f) contient une partie inclinée (284-f) continue, et
- chaque fente de flexibilité plus étroite (252-e₁, 282-e₁, 252-e₂, 282-e₂) contient deux parties inclinées (284-e₁, 284-e₂) continues, espacées par une section intacte (283-e) de paroi de tube (250), sur le côté postérieur (54) du tube (250).

14. Ensemble formant tige de pose (200) selon la revendication 13, dans lequel un angle, alpha, d'une inclinaison de chaque partie inclinée (284-e₁, 284-e₂, 284-f) est le même et est de 55 à 65 degrés.

15. Ensemble formant tige de pose (200) selon l'une quelconque des revendications 1 à 14, dans lequel la courbe de la section distale flexible (244) contient un rayon compris entre 30 et 50 mm.
